# EUROPEAN PATENT APPLICATION

(11) **EP 4 494 555 A1**
(43) Date of publication of application: **22.01.2025**
(21) Application number: 22958405.7
(22) Date of filing: 14.09.2022
(51) Int. Cl.: A61B 5/053

(54) **SIGNAL MEASUREMENT DEVICE**

(71) Applicant: Shenzhen Shokz Co., Ltd., Shenzhen, Guangdong 518108 (CN)
(72) Inventor: ZHOU, Xin, Shenzhen, Guangdong 518108 (CN); ZHANG, Yuxiang, Shenzhen, Guangdong 518108 (CN); SU, Lei, Shenzhen, Guangdong 518108 (CN); LI, Meiqi, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/118820
(87) International publication number: WO 2024/055212

(57) **Abstract**

Disclosed is a signal measuring device (100) including: at least one electrode (110) configured to contact with a human body to collect an electromyographic (EMG) signal of the human body in a target frequency range; an alternating current (AC) excitation source (120) electrically connected to the at least one electrode (110), the AC excitation source (120) being configured to provide an excitation signal at a first frequency to generate a detection signal reflecting a contact impedance between the at least one electrode (110) and the human body; and a gain circuit (130) configured to provide a gain for the EMG signal and the detection signal. In the present disclosure, by providing the gain circuit (130), gains are provided for the EMG signal and the detection signal collected so as to improve strengths and qualities of the EMG signal and the detection signal, and enable an accurate monitoring of the signal measuring device (100).

## Description

### TECHNICAL FIELD

The present disclosure relates to the field of circuit design, and in particular to a circuit structure of a signal measuring device.

### BACKGROUND

Signal measuring devices widely used in fields such as physiological monitoring, disease diagnosis, and experimental research are able to obtain data related to physical conditions of users by collecting physiological signals. For example, the signal measuring device may monitor and utilize information of electromyographic (EMG) signals to make clinical diagnoses on a wide range of disorders, such as muscular fatigue, myasthenia gravis, amyotonia, etc.

However, when physiological signals are collected by the signal measuring device, the contact impedance between at least one electrode and the human body becomes significant. This high impedance can arise from a poor fit between the at least one electrode and the human body, and near-insulating conditions of dry skin, or due to an unfulfillment of a movement of the human body. These factors can lead to weaker signal strength, which results in poor quality of the collected physiological signals, making accurate monitoring difficult.

Therefore, it is desired to propose a device capable of improving the quality of a physiological signal.

### SUMMARY

One of the embodiments of the present disclosure provides a signal measuring device. The device includes: at least one electrode configured to contact with a human body to collect an electromyographic (EMG) signal of the human body in a target frequency range; an alternating current (AC) excitation source electrically connected to the at least one electrode, the AC excitation source being configured to provide an excitation signal at a first frequency to generate a detection signal reflecting a contact impedance between the at least one electrode and the human body; and a gain circuit configured to provide a gain for the EMG signal and the detection signal.

In some embodiments, the target frequency range includes a range of 20 Hz-400 Hz, and the first frequency is not less than 250 Hz.

In some embodiments, a difference between the first frequency and any integer multiple of 50 Hz is not less than 1 Hz; or a difference between the first frequency and any integer multiple of 60 Hz is not less than 1 Hz.

In some embodiments, the first frequency is higher than the target frequency range.

In some embodiments, the gain of the gain circuit for the EMG signal is not less than 1/10 of the gain for the detection signal and is not greater than 10 times the gain for the detection signal.

In some embodiments, a frequency response of the gain circuit has one or more peaks in a first frequency range, the first frequency range being higher than 50 Hz and lower than the first frequency.

In some embodiments, a ratio of the gain of the gain circuit at 100 Hz to the gain at of the gain circuit 10 Hz is a first signal-to-noise ratio (SNR), and the first SNR is not less than 4.

In some embodiments, the ratio of the gain of the gain circuit at 100 Hz to the gain of the gain circuit at 50 Hz is a second SNR, and the second SNR is not less than 2.

In some embodiments, the gain circuit further includes a high-pass filtering circuit configured to filter the EMG signal and the detection signal.

In some embodiments, the gain circuit further includes a low-pass filtering circuit connected to the high-pass filtering circuit, and the low-pass filtering circuit is configured to filter high-frequency noises in the EMG signal and the detection signal; and the signal measuring device further includes an analog-to-digital converter (ADC) configured to perform an analog-to-digital conversion on the filtered EMG signal and the filtered detection signal.

In some embodiments, the ADC has a sampling rate greater than two times a third frequency, the third frequency being the greater of the first frequency and a frequency of the EMG signal.

In some embodiments, a ratio of the gain of the gain circuit at 100 Hz to the gain of the gain circuit at a second frequency is a third SNR, and the second frequency corresponds to 1/2 of a sampling rate of the ADC, and the third SNR is not less than 10.

In some embodiments, the high-pass filtering circuit includes an active bandpass filter configured to provide a first gain for the EMG signal and the detection signal; and the low-pass filtering circuit includes a passive low-pass filter coupled to the active bandpass filter.

In some embodiments, the high-pass filtering circuit includes a fifth-order high-pass filter, an input end of the fifth-order high-pass filter receives the EMG signal and the detection signal, and the fifth-order high-pass filter is further configured to provide a first gain for the EMG signal and the detection signal; and the low-pass filtering circuit includes a seventh-order low-pass filter coupled to the fifth-order high-pass filter, and the seventh-order low-pass filter is configured to provide a second gain for the EMG signal and the detection signal.

In some embodiments, the fifth-order high-pass filter and the seventh-order low-pass filter are Chebyshev filters.

In some embodiments, a secondary gain circuit is disposed between the at least one electrode and the high-pass filtering circuit, the secondary gain circuit provides a secondary gain, and the secondary gain is less than the first gain and the second gain.

In some embodiments, the at least one electrode includes a first electrode and a second electrode configured to collect a first EMG signal and a second EMG signal of the EMG signal, respectively; the AC excitation source includes two excitation sources connected to the first electrode and the second electrode, respectively, and provides two excitation signals to generate a first detection signal and a second detection signal of the detection signal, respectively; and the gain circuit includes a differential amplifier that performs differential amplification on the first EMG signal and the second EMG signal, and the first detection signal and the second detection signal, respectively.

In some embodiments, the gain circuit provides the gain to the EMG signal and the detection signal at the same time; or the gain circuit provides the gain to the EMG signal and the detection signal at different time periods, respectively.

In some embodiments, the signal measuring device collects the EMG signal and the detection signal at the same time; or the signal measuring device collects the EMG signal and the detection signal at different time periods, respectively.

In some embodiments, the signal measuring device is a wearable device.

In the embodiment of the present disclosure, by disposing the gain circuit to provide a gain for the collected EMG signal and the detection signal, so as to enhance the strength as well as the quality of the EMG signal and the detection signal, so that the signal measuring device may accurately perform monitoring.

### BRIEF DESCRIPTION OF THE DRAWINGS

The present disclosure will be further illustrated by way of exemplary embodiments, which will be described in detail with reference to the accompanying drawings. These embodiments are not limiting, and in these embodiments, the same counting denotes the same structure, wherein:
FIG. 1 is a block diagram illustrating a structure of a signal measuring device according to some embodiments of the present disclosure;
FIG. 2A is a diagram illustrating a frequency response curve of a signal measuring device according to some embodiments of the present disclosure;
FIG. 2B is a diagram illustrating a logarithmic frequency response curve of a signal measuring device according to some embodiments of the present disclosure;
FIG. 3 is a block diagram illustrating a structure of an exemplary signal measuring device according to some embodiments of the present disclosure;
FIGs. 4A-4B are schematic diagrams illustrating a structure of an exemplary high-pass filtering circuit according to some embodiments of the present disclosure;
FIGs. 5A-5B are schematic diagrams illustrating a structure of an exemplary low-pass filtering circuit according to some embodiments of the present disclosure;
FIG. 6 is a schematic diagram illustrating a structure of an exemplary secondary gain circuit according to some embodiments of the present disclosure;
FIG. 7A is a block diagram illustrating a circuit structure of a signal measuring device of Example 1 according to some embodiments of the present disclosure;
FIG. 7B is a schematic diagram illustrating the circuit structure of the signal measuring device of Example 1 according to some embodiments of the present disclosure;
FIG. 8A is a block diagram illustrating a circuit structure of an exemplary signal measuring device of Example 2 according to some embodiments of the present disclosure;
FIG. 8B is a schematic diagram illustrating the circuit structure of the exemplary signal measuring device of Example 2 according to some embodiments of the present disclosure;
FIG. 9 is a schematic diagram illustrating a circuit structure of a bandpass gain signal measuring device of Example 3 according to some embodiments of the present disclosure; and
FIG. 10 is a diagram illustrating frequency response curves of the signal measuring devices in FIG. 7B, FIG. 8B and FIG. 9.

### DETAILED DESCRIPTION

To more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for those skilled in the art to apply the present disclosure to other similar scenarios in accordance with these drawings without creative labor. Unless obviously obtained from the context or the context illustrates, otherwise, the same numeral in the drawings refers to the same structure or operation.

It should be understood that the terms "system," "device" as used herein, "unit" and/or "module" as used herein is a way to distinguish between different components, elements, parts, sections or assemblies at different levels. However, the words may be replaced by other expressions if other words accomplish the same purpose.

As shown in the present disclosure and the claims, unless the context clearly suggests an exception, the words "a," "an," "one," and/or "the" do not refer specifically to the singular, but may also include the plural. Generally, the terms "including" and "comprising" suggest only the inclusion of clearly identified steps and elements. In general, the terms "including" and "comprising" only suggest the inclusion of explicitly identified steps and elements that do not constitute an exclusive list, and the method or apparatus may also include other steps or elements.

Flowcharts are used in the present disclosure to illustrate operations performed by a system in accordance with embodiments of the present disclosure. It should be appreciated that the preceding or following operations are not necessarily performed in an exact sequence. Instead, steps can be processed in reverse order or simultaneously. Also, it is possible to add other steps to these processes or remove a step or steps from them.

A signal measuring device of one or more embodiments of the present disclosure may be applied in a device configured to collect one or more physiological signals, such as a smart wearable device, a medical detection device, or a signal analysis device. In some embodiments, the smart wearable device may be placed at various portions (e.g., calves, thighs, the waist, the back, the chest, shoulders, the neck, etc.) of a human body to collect physiological signals from a user is in different states, and the collected signals may be further processed. In some embodiments, the smart wearable device may include a smart bracelet, smart footwear, smart glasses, a smart helmet, a smartwatch, a smart garment, a smart backpack, a smart accessory, etc., or any combination thereof.

In some embodiments, the physiological signals are signals that can be detected and be able to reflect the state of the body, for example, the physiological signals may include respiratory signals, electrocardiogram (ECG) signals, electromyogram (EMG) signals, blood pressure signals, temperature signals, etc. In some embodiments, the physiological signals may be in a frequency range of 0.05 Hz ~2 kHz. The electrocardiographic signals may be in a frequency range of 0.05 Hz ~100 Hz, and the EMG signal may be in a frequency range of 10 Hz ~ 850 Hz.

In some embodiments, a strength of the physiological signals collected by the signal measuring device is affected by a variety of factors, such as a contact impedance between the human body and at least one electrode, an activation degree of a muscle of the human body, etc. In some embodiments, the contact impedance refers to an impedance generated by the skin of the human body contacting with the at least one electrode of the signal measuring device, and an impedance value may be affected by a state of dryness of the skin of the human body contacting with the electrode, and a state of the human body. For example, the drier the skin, the greater the impedance value of the contact impedance, resulting in a poorer quality of the collected physiological signals; conversely, the wetter the skin, the smaller the impedance value of the contact impedance, and the better the quality of the collected physiological signals. As another example, the poor contact between the electrode and the skin results in a greater impedance value of the contact impedance, making a quality of the physiological signals poorer. In some embodiments, the activation degree of the muscle is an active state of the muscle, which affects the strength of the EMG signal released by the muscle. For the user, there are a lot of daily exercise monitoring needs (e.g., walking, standing). Compared to fitness monitoring needs (e.g., squatting, running), a low activation degree of the muscle by daily exercise results in a low EMG strength, which affects subsequent recognition and processing.

In some embodiments, to improve the strength and the quality of the collected physiological signals (e.g., the EMG signal), a detection signal corresponding to the contact impedance may be obtained in advance, so as to detect the magnitude of the contact impedance to assess the quality of the physiological signals. In some embodiments, a gain may also be provided for the EMG signal and the detection signal, thereby improving the strengths of the EMG signal and the detection signal for subsequent high-resolution readout of the EMG signal. The following is a detailed description of the signal measuring device provided by the embodiments of the present disclosure, using the EMG signal as an example.

In some embodiments, if the user wears the at least one electrode incorrectly or the skin of the user is too dry during the collection of the EMG signal, the contact impedance between the human body and the at least one electrode is likely to be great, resulting in the strength of the EMG signal collected being too small, making it difficult to accurately perform physiology monitoring on the user. In some embodiments, if the activation degree of the muscle to be monitored during the signal collection process is insufficient, the strength of the EMG signal generated by the muscle is too small, making it difficult for the signal collection device to identify and process the EMG signal.

Thus, according to some embodiments of the present disclosure, firstly, the detection signal reflecting the contact impedance between the human body and the at least one electrode may be collected simultaneously with the collection of the EMG signal, so that the contact impedance may be subsequently adjusted to be appropriate and a high-quality EMG signal may be obtained. An appropriate gain is then provided for both the EMG signal and the detection signal to ensure subsequent high-resolution readout, identification, and processing of the EMG signal.

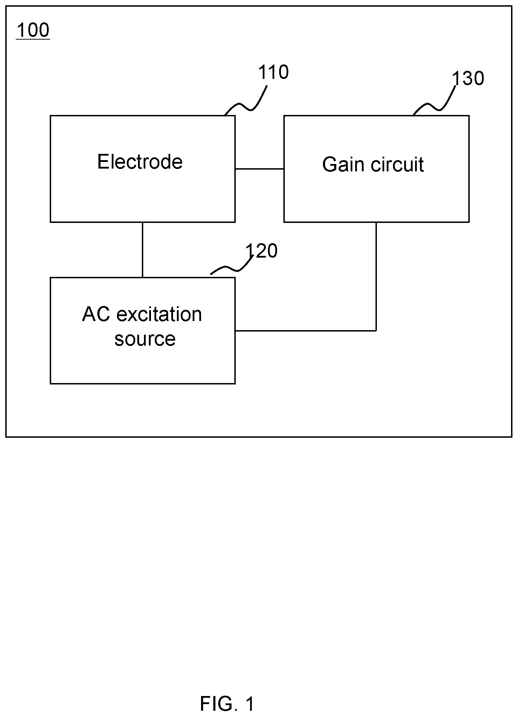

FIG. 1 is a block diagram illustrating a structure of a signal measuring device according to some embodiments of the present disclosure. As shown in FIG. 1, a signal measuring device 100 may include: an electrode 110, an alternating current (AC) excitation source 120, and a gain circuit 130. The electrode 110 is electrically connected to the AC excitation source 120, and the gain circuit 130 is electrically connected to the electrode 110 and the AC excitation source 120, respectively. In some embodiments, the electrode 110 may collect an EMG signal of a human body within a target frequency range; the AC excitation source 120 is configured to provide an excitation signal at a first frequency to generate a detection signal reflecting a contact impedance between the electrode 110 and the human body; and the gain circuit 130 is configured to provide a gain for the EMG signal and the detection signal.

The electrode 110 may be a circuit element that inputs or outputs current through contact. In some embodiments, the electrode 110 may be configured to contact with the human body to collect physiological signals (e.g., an EMG signal, an electrocardiograph (ECG) signal, a blood pressure signal, etc.) of the human body. In some embodiments, the electrode 110 may collect the EMG signal of the human body within the target frequency range. In some embodiments, the EMG signal is a physiological signal generated by a muscle of the human body, which reflects a state of neuromuscular activity of the human body. In some embodiments, the target frequency range may include frequencies of a majority of EMG signals generated by the muscle, and may also include a frequency of the EMG signal that is expected to be obtained.

In some embodiments, the detection signal corresponding to the electrode 110 may be generated under an external excitation (e.g., the excitation signal). The detection signal may reflect the contact impedance between the electrode 110 and the human body. In some embodiments, the detection signal may be a signal reflecting a magnitude of the contact impedance, and accordingly, may be configured to reflect the quality of the EMG signal. The quality of the EMG signal collected by a single electrode or a plurality of electrodes may be assessed by analyzing the detection signal corresponding to the single electrode, or a difference between the detection signals of the plurality of electrodes. For example, if the detection signal corresponding to a particular electrode indicates that the contact impedance of the electrode with the human body is greater than a certain threshold, it means that the electrode is not worn correctly or that the skin of the user is too dry. As another example, if a difference between the detection signals corresponding to two electrodes is great, it indicates that the difference between the contact impedances of the two electrodes with the human body is great, which means that at least one of the electrodes is not worn correctly, or the skin of the user is too dry, then the quality of the collected EMG signal is poor. Conversely, if the difference between the detection signals corresponding to the two electrodes is small, it indicates that the difference between the contact impedances of the two electrodes with the human body is small, which means that the quality of the collected EMG signal is good. In some embodiments, the detection signal may also reflect other information such as an electrode wearing state of the user, body composition information, a skin state, etc.

In some embodiments, the electrode 110 may be configured to contact with different portions of the human body to collect physiological signals of the different portions. For example, the electrode 110 may be configured to contact with portions such as the chest, the wrist, the back, legs, arms, the head, etc., of the user. In some embodiments, there may be one or more electrodes. For example, one electrode is configured to collect a signal of one portion, or two electrodes are used as a group to obtain the EMG signal of the same portion.

In some embodiments, the electrode may be in contact with the human body in the form of a flexible patch, which is a regular shape such as a circle, an oval, a rectangle, a diamond, or other irregular shapes. In some embodiments, the electrode may be made of a metal conductive material (e.g., copper, aluminum, nickel, chromium, alloys, etc.), a non-metal conductive material (e.g., a conductive plastic, a conductive rubber, a conductive silicone, a conductive fabric), or other conductive materials.

In some embodiments, the signal measuring device 100 may also include one or more reference ground electrodes (not shown in FIG. 1) that serve as a reference ground in the signal measuring device 100. In some embodiments, the one or more reference ground electrodes may be configured to contact with the human body to enable an excitation signal generated by the AC excitation source 120 to flow through the human body to the reference ground electrodes to form a closed loop circuit. In some embodiments, the one or more reference ground electrodes may be disposed in a flat and more stable (with less movement) portion of the body, such as the back, the head, etc., of the human body, which effectively reduces noise.

The AC excitation source 120 may be a circuit element that provides electrical energy. In some embodiments, the AC excitation source 120 may provide the excitation signal at the first frequency to generate the detection signal reflecting the contact impedance between the electrode 110 and the human body. In some embodiments, the AC excitation source 120 may be a current source or a voltage source. Merely by way of example, the excitation signal may be understood as forming the closed loop after flowing through the human body through the electrode 110, and the detection signal may correspond to a partial voltage of the contact impedance between the electrode 110 and the human body in the closed loop. The higher the strength of the AC excitation source, the easier the detection signal is detected. At the same time, it should be noted that the setting of the strength of the AC excitation source also needs to takes a safety voltage or a safety current of the human body into account to ensure the safety of the human body, and the strength of the AC excitation source should not be too high. In some embodiments, the current strength of the AC excitation source may be less than 1 mA. In some embodiments, the current strength of the AC excitation source may be less than 100 µA. In some embodiments, the current strength of the AC excitation source may be 10 µA.

In some embodiments, there may be only one AC excitation source 120, which is connected to a plurality of electrodes 110 through a plurality of branches to provide the excitation signals for the plurality of electrodes 110. Each branch may be provided with one electrode 110. In some embodiments, there may be a plurality of AC excitation sources 120, and each of the AC excitation sources 120 may be respectively connected to one or more electrodes 110, providing the excitation signal for each of the connected electrodes 110.

In some embodiments, the AC excitation source 120 may simultaneously generate a plurality of excitation signals with different frequencies and may provide the excitation signals to the plurality of electrodes 110 with the same frequency or different frequencies, thereby generating detection signals with different frequencies. For example, the AC excitation source 120 may provide the plurality of electrodes 110 with excitation signals with different frequencies to collect different detection signals. The plurality of electrodes 110 may have a greater distance (e.g., greater than or equal to 5 cm) from each other so that the closed loop through which the excitation signals flow may pass through different human tissues. In this way, the detection signals corresponding to the plurality of electrodes 110 with different frequencies may be configured to reflect the body composition information of the human body, such as body fat rate, bone density, body fluid content, etc. Specifically, the AC excitation source 120 may provide a second excitation signal at a frequency different from the first frequency to generate a second detection signal. The second detection signal may reflect impedance information on the closed loop formed between each electrode and the human body at another frequency (i.e., a frequency different from the first frequency), including the contact impedance between the electrode and the human body and the impedance of the human tissue on the closed loop. In some embodiments, the body composition information of the human body may be determined using a first detection signal generated by a first excitation signal (e.g., the excitation signal with the first frequency), and a second detection signal generated by a second excitation signal.

In some embodiments, the excitation signal may be a voltage signal or a current signal. In some embodiments, the excitation signal may be an AC signal with the first frequency so that the generated detection signal also has the first frequency. The first frequency may be configured in a frequency range that is less susceptible to external interference (e.g., an industrial frequency interference) and that interferes less with the EMG signal.

In some embodiments, a divider resistance may also be provided between the AC excitation source 120 and the electrode 110. As the electrode 110 and the divider resistance are in the same loop, the detection signal may correspond to a partial voltage of the contact impedance of the electrode 110 with the human body in the closed loop, or correspond to the partial voltage of the divider resistance in the closed loop. As the strength of the detection signal is affected by the contact impedance between the electrode 110 and the human body, the strength of the detection signal may reflect the magnitude of the contact impedance, which enables assessing the quality of the EMG signal. For example, the greater the strength of the detection signal corresponding to a certain electrode 110 indicates the greater the corresponding contact impedance, indicating that the quality of the EMG signal collected by that electrode is poorer; on the contrary, a smaller strength of the detection signal corresponds to a smaller contact impedance, indicating that the quality of the EMG signal collected by that electrode is better.

In some embodiments, a difference of the detection signals corresponding to the two electrodes may reflect a difference of the contact impedances with the human skin when the divider resistances of two electrodes have the same parameters. For example, the greater the difference between the detection signals of two branches, the greater the difference between the contact impedances corresponding to the two branches, and the worse the quality of the corresponding EMG signal; conversely, the smaller the difference between the detection signals of the two branches, the smaller the difference between the contact impedances corresponding to the two branches, and the better the quality of the corresponding EMG signal. In this way, by comparing the difference between the detection signals, whether a fitting state between the two electrodes and the human body is good or not may be reflected, and thus whether the EMG signal collected by the electrodes satisfies a requirement may be reflected.

In some embodiments, the target frequency range may be set based on a frequency range of the EMG signal expected to be collected. In some embodiments, the target frequency range may be a range of 10 Hz-850 Hz. In some embodiments, the target frequency range may be a range of 20 Hz-400 Hz. In some embodiments, the first frequency may be set based on the frequency range of the EMG signal expected to be collected. In some embodiments, the first frequency may be no less than 250 Hz.

In some embodiments, the first frequency of the excitation signal may be set according to the target frequency range. In some embodiments, the first frequency may be higher than the target frequency range to avoid a great portion of frequency bands where the EMG signals are located and reduce mutual interference between the EMG signals and the excitation signals. In some embodiments, the first frequency may be no less than 850 Hz. In some embodiments, the first frequency may be not less than 400 Hz. If the strength of the excitation signal is greater or the contact impedances corresponding to the electrodes can generate enough voltage, the interference of the EMG signal to the excitation signal is lower, and the excitation signal (e.g., the excitation signal whose first frequency is not less than 400 Hz) may tolerate a small portion of the EMG signals (e.g., the EMG signals at frequencies within a range of 400 Hz-850 Hz).

As there is also the interference from an industrial frequency (e.g., 50Hz or 60Hz AC power supply and harmonics thereof) noise in the signal measuring device 100, in some embodiments, the first frequency may also be set according to a frequency range in which the industrial frequency noise is located. For example, the set first frequency may avoid effects of the industrial frequency noise to circumvent the industrial frequency (IF) and a harmonic noise interference thereof. In some embodiments, the difference between the first frequency and any integer multiple of 50 Hz may be no less than 1 Hz, or, the difference between the first frequency and any integer multiple of 50 Hz may be no less than 2%. In some embodiments, the difference between the first frequency and any integer multiple of 60 Hz may be no less than 1 Hz, or, the difference between the first frequency and any integer multiple of 60 Hz may be no less than 2%.

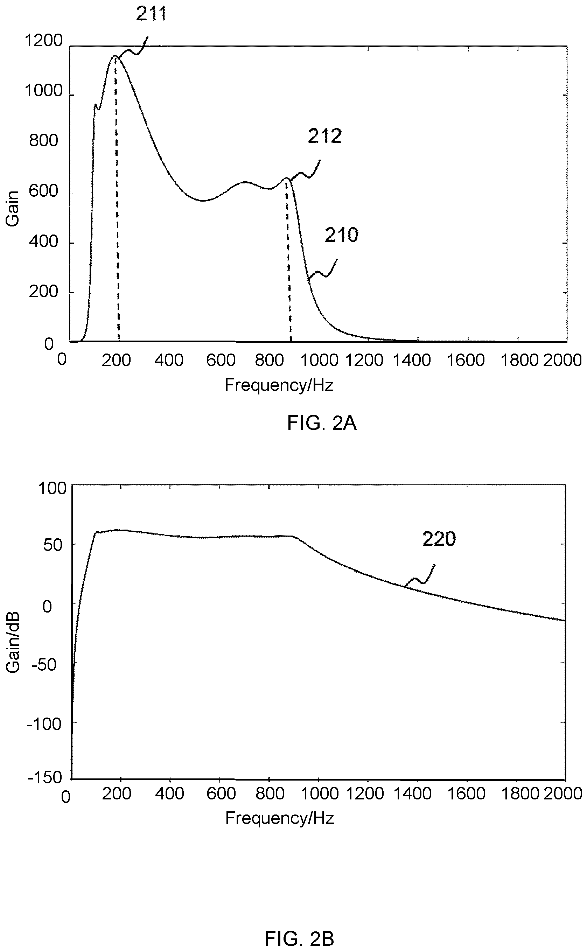

It should be noted that during a signal measurement, there are a great count of interfering signals in a low frequency band due to movements of the head, limbs, jaw, and tongue such as tiny peristalsis and swallowing, i.e., motion artifacts (MA). The interference of the MA may be avoided by setting the first frequency of the excitation signal to avoid the lower frequency. In some embodiments, the first frequency may also be set based on a sampling frequency of a subsequent processing circuit, preventing the sampling frequency provided by the processing circuit from being unable to realize an effective sampling of the detection signals, so as to ensure the quality of the signals. For a specific description of the relationship between the sampling frequency and the first frequency, please refer to other portions of the present disclosure, for example, FIGs. 2A-2B and the descriptions thereof.

The gain circuit 130 is a signal processing circuit that amplifies the signal. In some embodiments, the gain circuit 130 may be configured to provide a gain for the EMG signal and the detection signal. For example, the EMG signal and the detection signal may be used as inputs to the gain circuit 130, and the EMG signal and the detection signal may be amplified by the gain circuit 130, thereby increasing the strengths and SNRs of the EMG signal and the detection signal, and improving the quality of the signals.

It should be noted that the contact impedance between the human body and the electrodes may have a small value at the first frequency, which results in a low strength of the detection signal generated by the voltage division, which makes it difficult for subsequent recognition and process of the detection signal. In the embodiment of the present disclosure, by providing the gain for the EMG signal and the detection signal at the same time through the gain circuit 130, the strengths of the EMG signal and the detection signal may be improved, and subsequent accurate recognition and processing of the EMG signal and the detection signal may be ensured.

In some embodiments, the gain circuit 130 may provide a gain for a full-band signal, or may provide a gain for a partial-band signal. Taking the EMG signal as an example, since most of the EMG signals are distributed in a frequency band of 10 Hz-850 Hz, the gain circuit 130 may mainly provide a gain for the EMG signals in the frequency band of 10 Hz-850 Hz, and avoid providing a gain for other frequency bands (e.g., other than the frequency band of 10 Hz-850 Hz), so as to inhibit the interference of the noise on the EMG signals and to improve the SNR of the EMG signal.

In some embodiments, the gain may be expressed as a degree of amplification of the EMG signal and the detection signal. For example, the greater the gain provided by the gain circuit 130, the greater magnification of the EMG signal and the detection signal are amplified, and conversely, the smaller the gain provided by the gain circuit 130, the smaller the magnification of the EMG signal and the detection signal are amplified.

In some embodiments, the magnitude of the gain may be set based on an actual signal strength and an expected signal strength. In some embodiments, the magnitude of the gain may be set based on the actual signal strengths and the expected signal strengths of the EMG signal and the detection signal. For example, as the actual signal strengths of the EMG signal and the detection signal are about 10µV or below, and a resolution of a commonly used 12-bit analog-to-digital converter (ADC) is about 0.8mV (i.e., the expected signal strength) under a 3.3V power supply, then the gain circuit 130 may be utilized to provide gains of more than 100 to 1,000 times, so that the signal strengths of the EMG signal and the detection signal after the gain may reach 1mV~10mV.

In some embodiments, the gain circuit 130 may provide the same or different gains for the EMG signal and the detection signal, respectively. In some embodiments, the gained EMG signal and the detection signal may be filtered, etc., by a same circuit. To prevent the difference in gains from being too great, resulting in a too-great difference in the strengths of the gained EMG signal and the detection signal, in some embodiments, the gain of the gain circuit 130 for the EMG signal may be no less than 1/10 of the gain for the detection signal, and may be no greater than 10 times the gain for the detection signal. That is to say, a relative value between the gain provided to the EMG signal and the gain provided to the excitation signal may be set to within 10 times of each other, so as to ensure that the strengths of the EMG signal and the detection signal after the gain are not much different from each other, which facilitates subsequent identification and processing.

As the gain circuit 130 needs to provide a gain for both the EMG signal and the excitation signal, the frequency response of the circuit needs to gain the EMG frequency band in addition to an excitation source band, with an adequate suppression on frequencies lower than the EMG frequency and higher than the excitation frequency.

In some embodiments, the frequency response of the gain circuit 130 may have one or more peaks in a first frequency range. The first frequency range herein includes the frequency of the EMG signal and the frequency of the detection signal. For example, the first frequency range may be a frequency range above 50 Hz and below the first frequency. As the gain circuit 130 needs to provide a gain for the EMG signal and the detection signal, respectively, in some embodiments, according to the frequency of the EMG signal and the detection signal, a count of peaks of the frequency response of the gain circuit 130 may be determined. In some embodiments, the frequency response of the gain circuit 130 may be bimodal, with two peaks being proximate to the target frequency range of the EMG signal and the first frequency, respectively. In some alternative embodiments, the frequency response of the gain circuit 130 has only one peak that has a broader bandwidth. The frequency response has a higher gain on the frequencies in the first frequency range than on frequencies in other bands, thereby suppressing signals at frequencies lower than the frequency of the EMG signal and/or higher than the frequency of the excitation signal, reducing the interference to the EMG signal and the excitation signal.

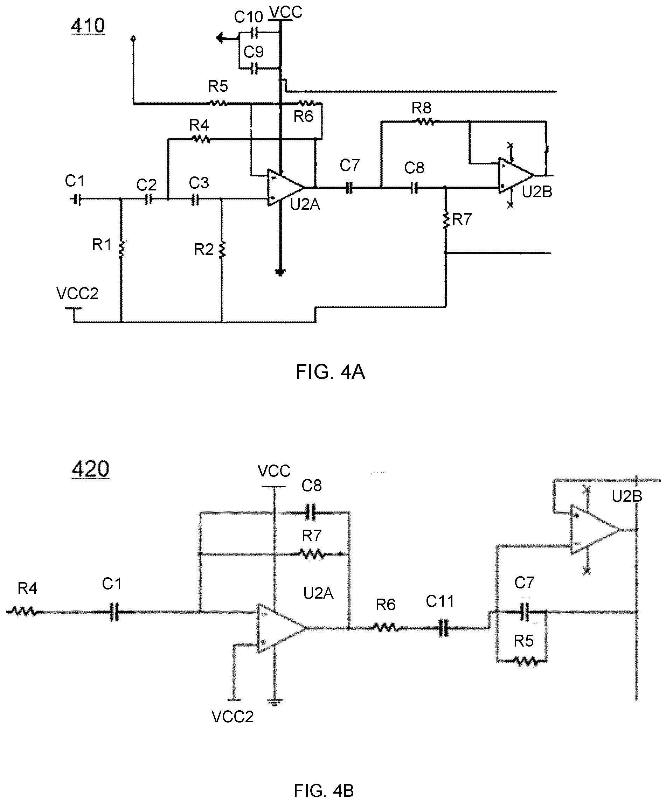

Taking the example of the AC excitation source providing the excitation signals of two frequencies (e.g., the aforementioned first excitation signal and the second excitation signal), if the frequencies of the first excitation signal and the second excitation signal are 620Hz and 820Hz, respectively, the frequency response of the gain circuit 130 may be designed to have a peak with a width covering 51 Hz-820Hz within the first frequency range, or it may be designed to have two peaks covering 51 Hz-400Hz and 620Hz-820Hz, respectively, or, to have three peaks with widths covering 51 Hz-400Hz, near 620Hz, and near 820Hz, respectively, in the first frequency range, so as to ensure that a great gain is provided for the EMG signal and for the detection signal in the first frequency range. For the structure of the gain circuit 130 for realizing different frequency responses, please refer to other portions of the present disclosure, for example, FIGs. 4A-6 and related descriptions thereof.

FIG. 2A is a diagram illustrating a frequency response curve of a signal measuring device according to some embodiments of the present disclosure, and FIG. 2B is a diagram illustrating a logarithmic frequency response curve of a signal measuring device according to some embodiments of the present disclosure.

Referring to FIGs. 2A-2B, a curve 210 is bimodal, and a curve 220 obtained by translating the curve 210 into logarithmic coordinates is in a bandpass shape with a flatter passband. The curve 210 has two peaks (a peak 211 and a peak 212 as shown in FIG. 2A), with the peak 211 distributed near 183 Hz, which corresponds to a gain of 1160 times, and the peak 212 distributed near 873 Hz, which corresponds to a gain of 664.3 times. According to the curve 210, the gain circuit 130 may provide a gain of greater than 900 times for an EMG signal in a frequency range of about 100 Hz-300 Hz, and provide a gain of 100 times-700 times for a signal in a frequency range of about 800 Hz-1000 Hz (e.g., a detection signal may be set to 820 Hz). In some embodiments, the gain requirement may be determined based on the strength of the detection signal, thereby adjusting the gain provided by the gain circuit 130. For example, the gain provided by the gain circuit 130 may be adjusted from (133 times, 1000 Hz) to (664.3 times, 873 Hz).

There are a lot of interfering signals during a signal measurement process, such as the aforementioned MA, IF and its harmonic, and a mixed noise, etc. On one hand, if an excessive gain is provided for the aforementioned interfering signals, the interfering signals after the gain may exceed a measurement range of the circuit, which leads to a saturation of the circuit and a loss of data, making it difficult to restore data through an algorithm. On the other hand, if the excessive gain is provided for the high- frequency noise, due to a limitation of a sampling rate, the high-frequency noise that exceeds the sampling rate may be intermixed, which raises background noise, so that the high-frequency noise is overlapped with the EMG signal, making it difficult for distinction and process.

To suppress the gain provided by the gain circuit 130 to the foregoing interfering signals, in some embodiments, a gain SNR may be adjusted to limit the gain provided by the gain circuit 130 on the frequencies corresponding to the interfering signals. The gain SNR may be a ratio of the gain at the frequency of the EMG signal to the gain at the frequency of the interfering signal. In some embodiments, the gain SNR may include one or more of an MA SNR, an IF SNR, and a mixed SNR. The foregoing three gain SNRs are described below, respectively.

In some embodiments, the gain at 10 Hz may be represented as the gain of the MA, and a ratio of the gain at 100 Hz by the gain circuit 130 to the gain at 10 Hz may be a first SNR (i.e., the aforementioned MA SNR). To improve the gain SNR of the gain circuit 130, this first SNR can be set to be not less than 4. Taking the curve 210 shown in FIG. 2A as an example, a gain at 100 Hz is about 940 times, a gain at 10 Hz is about 0.00186 times, and the first SNR may be 505376.

In some embodiments, the gain at 50 Hz may be represented as the gain of the IF noise, and a ratio of the gain at 100 Hz to the gain at 50 Hz by the gain circuit 130 is a second SNR (i.e., the aforementioned IF SNR). To improve the gain SNR of the gain circuit 130, the second SNR may be not less than 2. Taking the curve 210 shown in FIG. 2A as an example, a gain at 100 Hz is of about 940 times, a gain at 50 Hz is about 8.26 times, and the second SNR may be 114.

In some embodiments, as a generation of the mixed noise is related to the sampling rate, then 1/2 of the frequency of the sampling rate of the ADC may be expressed as the frequency of the mixed noise, which is also referred as to a second frequency. For example, the frequency of the mixed noise may be 2 kHz when the sampling frequency is 4 kHz. The ratio of the gain at 100 Hz to the gain at the second frequency by the gain circuit 130 may be a third SNR (i.e., the aforementioned mixed SNR). To improve the gain SNR of the gain circuit 130, the third SNR may be not less than 10. Taking the curve 210 shown in FIG. 2 A as an example, the gain at 100 Hz is about 940 times, the gain at 2 kHz is about 0.185 times, then the third SNR may be 5081.

It should be noted that the gain SNR may also be used to measure the quality of the gain circuit, and a higher gain SNR of the signal measuring device may indicate a better strength and quality of the obtained EMG signal, and that the gain circuit is of higher quality.

In the embodiment of the present disclosure, an excessive gain for the interference signal may be avoided by adjusting the gain SNR, so as to avoid a loss of data due to the gained interference signal exceeding a measurement range of the circuit, and a possibility for overlapping between a high-frequency noise and the EMG signal may also be reduced, so as to improve the quality of the EMG signal and the detection signal for subsequent high-resolution readout of the EMG signal and the detection signal.

In some embodiments, the gain circuit 130 may provide a gain for both the EMG signal and the detection signal. Correspondingly, in some embodiments, the signal measuring device 100 may collect the EMG signal and the detection signal at the same time. That is, the signal measuring device 100 may receive both the EMG signal and the detection signal as input signals and provide a gain for the input signals at the same time. In this way, the detection signal may reflect the contact impedance between a human body and at least one electrode in real time, so that the quality of the EMG signal may be determined in a timely manner.

In some embodiments, the signal measuring device 100 may obtain the EMG signal and the detection signal at different time periods, respectively. Correspondingly, the gain circuit 130 may provide the gain for the EMG signal and the detection signal, respectively, at different time periods. Exemplarily, the signal measuring device 100 may collect the EMG signal during a first time period and provide a gain for the EMG signal through the gain circuit 130. Exemplarily, the signal measuring device 100 may collect the detection signal during a second time period and provide a gain to the detection signal through the gain circuit 130. For example, the signal measuring device 100 may include a switching circuit. The switching circuit may be connected to the electrode 110, and the switching circuit may be configured to control a conductive state between the electrode 110 and the gain circuit 130, and may also be configured to control the conductive state between the electrode 110 and the AC excitation source 120, so that at a same moment, only the electrode remains in the electrically conductive state with the gain circuit 130, or only the electrode 110 remains in an electrically conductive state with the AC excitation source 120. In this way, by performing a time dividing collection and a processing on the detection signal and the EMG signal, a processing load of the device may be reduced, and computational resources may be saved while avoiding the signal interfering with each other.

It may be noted that in some other embodiments, the signal measuring device 100 may collect the EMG signal and the detection signal in different time periods, but use the gain circuit 130 to provide a gain for the EMG signal and the detection signal. The signal measuring device 100 may also collect the EMG signal and the detection signal at the same time, then use the gain circuit 130 to provide a gain for the EMG signal and the detection signal at the same time. In some embodiments, the selection of collecting and processing the signals in different time periods or at the same time may be performed based on signal quality and device resources.

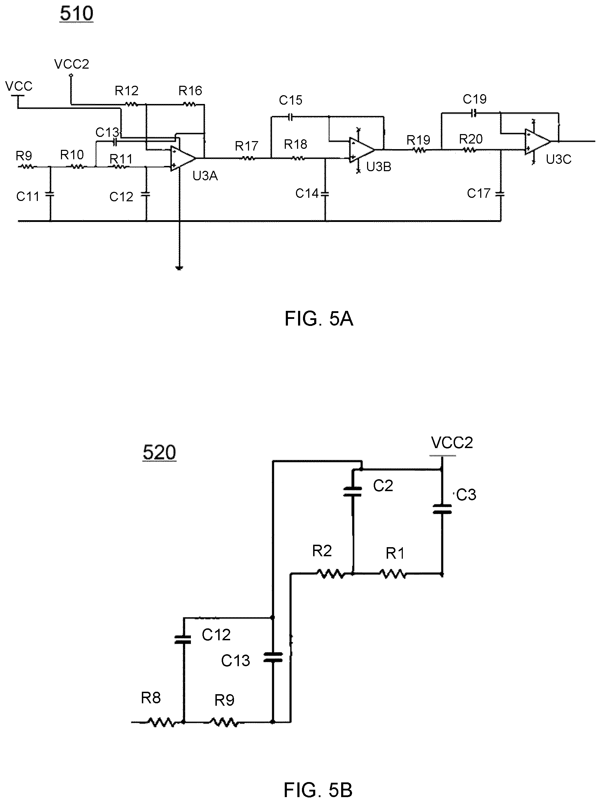

In some embodiments, the gain circuit 130 may include a multi-stage amplification subcircuit for performing a multi-stage amplification processing on the input signals (e.g., the EMG signal and the detection signal) to achieve a function of providing gain. In some embodiments, the amplification subcircuit may be provided independently in the signal measuring device 100, such as a secondary gain circuit mentioned elsewhere in the present disclosure. In some embodiments, the amplification subcircuit may also be provided as a component in a circuit with other functions. In this way, while realizing other functions, a gain may be provided, such as the gain provided by a high-pass filtering circuit and a low-pass filtering circuit elsewhere in the present disclosure, etc. In some embodiments, different stages of the multi-stage amplification subcircuit may have different amplification gains for the input signal. For example, the gain of the amplification subcircuit located in a former stage (e.g., a secondary gain provided by a secondary gain circuit) may be less than the gain located in a later stage (e.g., a first gain provided by the high-pass filtering circuit 140 or a second gain provided by the low-pass filtering circuit 150). For a specific structural description of the amplification subcircuit, please refer to other portions of the present disclosure, e.g., FIGs. 5A-6 and the descriptions thereof.

In some embodiments, the gain circuit 130 may also perform a signal processing on the EMG signal and the detection signal.

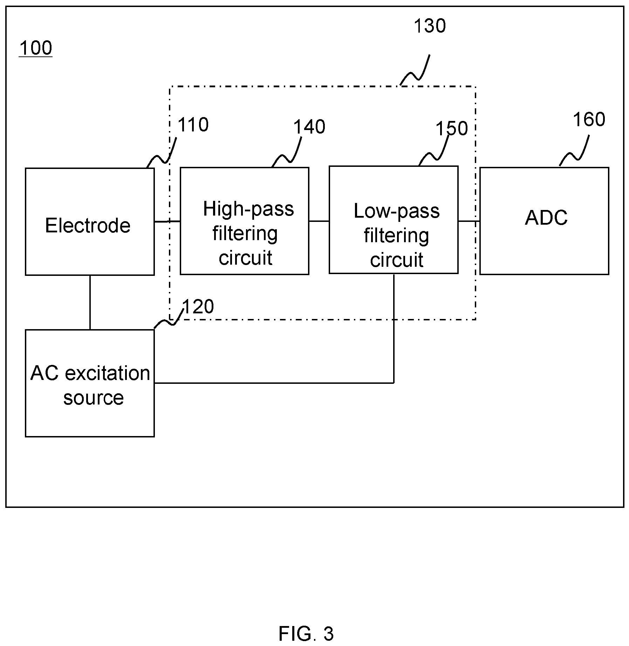

In some embodiments, the gain circuit 130 refers to a structural module with a certain signal processing function, such as an amplification, a rectification, an A/D conversion, filtering, a logic processing, etc. In some embodiments, the gain circuit 130 may include a processor (not shown in FIG. 3), such as a digital signal processor (DSP) chip, a central processing unit/processor (CPU), a microcontroller unit (MCU), etc.

Merely as an example, in some embodiments, the gain circuit 130 may individually process the detection signal corresponding to a particular electrode and evaluate the EMG signal collected by that electrode. Exemplarily, the gain circuit 130 may amplify the detection signal corresponding to the electrode 110, and subsequently, the ADC may be configured to perform an analog-to-digital (A/D) conversion. The detection signal may individually reflect the contact impedance between the electrode 110 and the human body, and thus reflect a fitting state of the electrode 110 and the human body, so as to determine the quality of the EMG signal.

In some embodiments, the processor may combine the detection signals corresponding to the plurality of electrodes to comprehensively evaluate the EMG signals collected by the electrodes. Exemplarily, the processor may perform an operational amplification (e.g., a differential amplification) on the detection signals corresponding to the plurality of electrodes 110 and then perform the A/D conversion on an amplified result. The detecting signal after the differential amplification may reflect a difference in the contact impedance between the two electrodes and the human body, and thus reflect whether both the two electrodes fit the human body well.

In some embodiments, the gain circuit 130 may filter the detection signal corresponding to the electrode 110 to distinguish the EMG signal collected by the electrodes 110. In some embodiments, the gain circuit 130 may directly determine the difference between the detection signals corresponding to the first electrode 111 and the second electrode 112, and then evaluate the EMG signal based on the difference. In some embodiments, the evaluation of the EMG signal by the gain circuit 130 may include determining the quality of the EMG signal and evaluating the fitting state between the one or more electrodes 110 and the human body. In some embodiments, the gain circuit 130 may also determine whether to output the EMG signal based on an evaluation result of the EMG signal. In some embodiments, the gain circuit 130 may also output a user instruction based on the evaluation result of the EMG signal to instruct the user to perform instructions such as adjusting a wearing state of the electrodes or instructing the user to continue warming up to improve the quality of the subsequently collected EMG signal. In some embodiments, the gain circuit 130 may also perform the signal processing on the EMG signal by a signal processing algorithm (e.g., a signal filtering, a wavelet transform, a machine learning) to improve the quality of the EMG signal.

In some embodiments, the gain circuit 130 may also include a transceiver circuit, such as a voice input/output circuit, a display circuit, a wireless/wired communication circuit, etc., and the evaluation result of the gain circuit 130 on the EMG signal collected by the aforementioned electrodes may be output through the transceiver circuit. In some embodiments, the gain circuit 130 may send the EMG signal and/or the evaluation result of the EMG signal to other ends and output the EMG signal and/or the evaluation result (e.g., through a display screen or a speaker broadcast) in wired or wireless (e.g., Bluetooth, WiFi, etc.) means.

It is noted that the transceiver circuit may be served as a circuit component within the gain circuit 130 and may be connected to the processor to transmit data and signals; or the transceiver circuit may be provided integrally within the processor to allow the processor to, through built-in communication components, realize the input and output functions.

In some embodiments, the gain circuit 130 may evaluate the human body state based on the plurality of detection signals at different frequencies. Exemplarily, the gain circuit 130 may determine body composition information reflecting the state of the human body, such as body fat percentage, bone density, body fluid content based on the plurality of detection signals at different frequencies.

It is noted that processing functions of the gain circuit 130 may include, but are not limited to, the above listed contents. For example, in some embodiments, the gain circuit 130 may also determine a sum of the detection signals corresponding to the plurality of electrodes 110, and then evaluate the foregoing physiological signals based on the sum. In some situations, considering that the evaluation of the EMG signal based on the difference of the detection signals (or the value after the differential amplification by an operational amplifier) is able to better reflect the quality of the EMG signal than the evaluation based on the sum of the detection signals, the signal measuring device 100 is mainly described in the embodiments of the present disclosure based on the difference of the detection signals (or the value after the differential amplification by the operational amplifier) as an example.

FIG. 3 is a block diagram illustrating a structure of an exemplary signal measuring device 100 according to some embodiments of the present disclosure. As shown in FIG. 3, the gain circuit 130 may also include the high-pass filtering circuit 140, which is connected to the electrode 110.

The high-pass filtering circuit 140 may be a circuit structure that performs a high-pass filtering on a signal. In some embodiments, the high-pass filtering circuit 140 may be configured to filter out an interfering signal with a lower frequency (e.g., low frequency noises including an MA, an IF noise, etc.) from an EMG signal and a detection signal. In some embodiments, the high-pass filtering circuit 140 may allow a signal above a first cutoff frequency (e.g., the EMG signal and the detection signal) to pass through, and prevent the interfering signal below or equal to the first cutoff frequency from passing through, thereby enabling the filtering of the signal. In some embodiments, the first cutoff frequency may be set based on the frequencies of the EMG signal and the detection signal, or be set based on the frequencies of the low frequency noises expected to be filtered out. In some embodiments, the first cutoff frequency may be less than or equal to a frequency in a target frequency range and the lowest frequency in the first frequency range. Exemplarily, if the target frequency range corresponding to the EMG signal is a range of 20 Hz-400 Hz and the first frequency of the detection signal is 250 Hz, the first cutoff frequency may be less than or equal to 20 Hz. In some embodiments, the first cutoff frequency may be greater than or equal to a frequency of the MA and the highest IF. If the frequency of the MA, the IF, and the harmonics thereof is in a range of 10 Hz-50 Hz, then the first cutoff frequency may be greater than 50 Hz.

In some embodiments, the high-pass filtering circuit 140 may be replaced by a bandpass filtering circuit. Similarly, a bandwidth frequency range of the bandpass filtering circuit may be related to the frequencies of the EMG signal and the detection signal, or related to the frequencies of the low frequency noise. For example, the bandwidth frequency range of the bandpass filtering circuit may include frequencies in the target frequency range and the first frequency, and the bandwidth frequency range may have no intersection with the frequency range of the MA and with the frequency range of the IF noise.

In the embodiment of the present disclosure, by setting the high-pass filtering circuit 140, the low frequency noises such as the MA, the IF and the harmonics thereof may be filtered out, so as to avoid the gain circuit 130 from providing a gain for the low frequency noises of greater strengths, and ensure that the gained signal does not exceed a measurement range.

In some embodiments, an amplification subcircuit in the high-pass filtering circuit 140 (not shown in FIG. 3) may also provide a first gain for the EMG signal and the detection signal to achieve a gain function. In some embodiments, the first gain may be a partial gain provided for the EMG signal and the detection signal. In some embodiments, a magnitude of the first gain may be determined by adjusting component parameters (e.g., a value of a capacitor resistor, etc.) in the high-pass filtering circuit 140.

Several examples of the high-pass filtering circuit 140 are provided below to describe in detail the specific implementation of the high-pass filtering circuit 140.

FIGs. 4A-4B are schematic diagrams illustrating a structure of an exemplary high-pass filtering circuit 140 according to some embodiments of the present disclosure. In some embodiments, the high-pass filtering circuit 140 may include a fifth-order high-pass filter 410, and an input end of the fifth-order high-pass filter 410 is coupled to the electrodes 110 to take an EMG signal and a detection signal as inputs for filtering.

As shown in FIG. 4A, the fifth-order high-pass filter 410 may include an operational amplifier U2A-U2B, resistors R1-R2, R4-R8, and capacitors C1-C3, C7-C10. A positive phase input end of the operational amplifier U2A may be connected to the series-connected capacitors C1-C3. The positive phase input end of the operational amplifier U2A may also be connected to a power supply VCC2 through the resistor R2. A negative phase input end of the operational amplifier U2A may be connected to an output end of the operational amplifier U2A through the resistor R6, and the negative phase input end of the operational amplifier U2A may also be connected to the power supply VCC2 through the resistor R5. A power supply end of the operational amplifier U2A may be connected to the power supply VCC, and a grounding end of the operational amplifier U2A may be grounded. A positive phase input end of the operational amplifier U2B may be connected to the output end of the operational amplifier U2A through the series-connected capacitors C7-C8, the negative phase input end of the operational amplifier U2B may be connected to the output end of the operational amplifier U2B, and the power supply end of the operational amplifier U2B may be connected to the grounding end.

In some embodiments, the fifth-order high-pass filter 410 is also configured to provide a first gain for the EMG signal and the detection signal. In some embodiments, the operational amplifier U2A and the operational amplifier U2B may provide a first gain for the input signals (e.g., the EMG signal and the detection signal). A size of the first gain may be related to parameters of the resistors R1-R2, R4-R8 and the capacitors C1-C3, C7-C8. In some embodiments, the first gain may be provided after the fifth-order high-pass filter 410 finished filtering the EMG signal and the detection signal. In some embodiments, the output end of the operational amplifier U2B may be configured to output the EMG signal and the detection signal after the high-pass filtering and a partial gain.

One end of the resistor R1 may be connected to a connection point between the capacitor C1 and the capacitor C2, and the other end of resistor R1 may be connected to a virtual ground VCC2 to boost a voltage of the circuit, so that the EMG signal and the detection signal are greater than 0 V for subsequent readout using an ADC. One end of the resistor R4 may be connected to the connection point between the capacitor C2 and the capacitor C3, and the other end of the resistor R4 may be connected to the output end of the operational amplifier U2A. One end of the resistor R7 may be connected to the positive phase input end of the operational amplifier U2B, and the other end of the resistor R7 may be connected to the virtual ground VCC2. One end of the resistor R8 may be connected to the negative phase input end of the operational amplifier U2B, and the other end of the resistor R8 may be connected to the positive phase input end of the operational amplifier U2B. The capacitor C10 and the capacitor C9 are connected in parallel, with one end of both connected to the power supply VCC and the other end grounded. The capacitor C10 and the capacitor C9 may be configured to filter clutter waves from the power supply VCC.

In some embodiments, the fifth-order high-pass filter 410 may use the operational amplifier U2A and the operational amplifier U2B for the high-pass filtering of the detection signal and the EMG signal. In some embodiments, the first cutoff frequency of the fifth-order high-pass filter 410 may be determined by adjusting component parameters of the operational amplifier U2A and the operational amplifier U2B. In some embodiments, the fifth-order high-pass filter 410 may be a Chebyshev filter. The Chebyshev filter decays faster than other filters (e.g., a Butterworth filter) in a transition band, and a frequency response curve of the Chebyshev filter is closer to the frequency response curve of an ideal filter, which provides a better filtering effect.

In some embodiments, the high-pass filtering circuit 140 may include an active bandpass filter 420, and an input end of the active bandpass filter 420 may be coupled to the electrodes 110 to filter the EMG signal and the detection signal as inputs.

As shown in FIG. 4B, the active bandpass filter 420 may include the operational amplifiers U2A-U2B, the resistors R4, R6-R7, and the capacitors C1, C7-C11. The positive phase input end of the operational amplifier U2A may be connected to the virtual ground VCC2. The negative phase input end of the operational amplifier U2A may be connected to the series-connected capacitor C1 and the resistor R4, and the negative phase input end of the operational amplifier U2A may be connected to output end of the operational amplifier U2A through the parallel-connected capacitor C8 and the resistor R7. The power supply end of the operational amplifier U2A is connected to the power supply VCC, and the grounding end of the operational amplifier U2A may be grounded. The negative phase input end of the operational amplifier U2B may be connected to the output end of the operational amplifier U2A through the series-connected resistor R6 and capacitor C11, and the positive input end of the operational amplifier U2B may be connected to the virtual ground VCC2. The negative phase input end of the operational amplifier U2B may be connected to the output end of the operational amplifier U2A through the capacitors C7 and R5 connected in parallel, and the power supply end and the grounding end of the operational amplifier U2B may be connected.

In some embodiments, similar to the high-pass filter 410 described above, the active bandpass filter 420 is also configured to provide a first gain for the EMG signal and the detection signal. In some embodiments, the operational amplifier U2A and operational amplifier U2B of the active bandpass filter 420 may provide the first gain for the input signals (e.g., the EMG signal and the detection signal). A size of the first gain may be related to parameters of the resistors R4, R7, and the capacitors C1, C8. In some embodiments, the first gain may be provided after the active bandpass filter 420 has filtered the EMG signal and the detection signal.

In some embodiments, similar to the high-pass filter 410 described above, the output end of the operational amplifier U2B of the active bandpass filter 420 may be configured to output the EMG signal and the detection signal that have been bandpass filtered and partially gained. The capacitor C10 and the capacitor C9 are connected in parallel, with one end of both connected to the power supply VCC and the other end grounded.

In some embodiments, the active bandpass filter 420 may bandpass filter the EMG signal and the detection signal through the resistors R4, R5-R7, and the capacitors C1, C7-C8, C11. In some embodiments, a bandwidth frequency range of the active bandpass filter 420 may be determined by adjusting the component parameters of the aforementioned resistors R4, R5-R7, and the capacitors C1, C7-C8, C11.

Continuing to refer to FIG. 3, in some embodiments, the gain circuit 130 may include: the low-pass filtering circuit 150 connected to the high-pass filtering circuit 140, and the low-pass filtering circuit 150 is configured to filter the high-frequency noises of the EMG signal and the detection signal.

The low-pass filtering circuit 150 may also be a circuit structure that performs low-pass filtering on a signal. In some embodiments, the low-pass filtering circuit 150 may be configured to filter the interfering signals with higher frequencies in the EMG signal and the detection signal, for example, a high-frequency noise such as a mixed noise. In some embodiments, the low-pass filtering circuit 150 may allow the signals below a second cutoff frequency (e.g., the EMG signal and the detection signal) to pass through, so as to prevent the interfering signals above or equal to the second cutoff frequency from passing through, thereby enabling the filtering of the signals. Similar to the above-described high-pass filtering circuit 140, in some embodiments, the second cutoff frequency may be set based on the frequencies of the EMG signal and the detection signal, or may be set based on the frequency of the high-frequency noise expected to be filtered. In some embodiments, the second cutoff frequency may be greater than or equal to a frequency in a target frequency range and the highest frequency in the first frequency. In some embodiments, the second cutoff frequency may be less than or equal to 1/2 of a sampling rate.

In the embodiment of the present disclosure, by sequentially setting up a signal processing architecture of the high-pass filtering and the low-pass filtering, and by providing the gain in the process of the high-pass filtering and the low-pass filtering, on the one hand, it is possible to avoid providing gain for the low frequency signals with greater strength while suppressing the low frequency noises timely, so as to prevent the gained signal from exceeding the measurement range and leading to a data loss, and to improve a stability of the signal collection; on the other hand, by gaining the architecture of the low-pass filtering, the high-frequency noise introduced during the signal amplification may be filtered, so as to avoid the introduction of the mixed noise in the subsequent ADC process.

In some embodiments, an amplification subcircuit in the low-pass filtering circuit 150 may also provide a second gain for the EMG signal and the detection signal to achieve a gain function. In some embodiments, the second gain may be a partial gain provided to the EMG signal and the detection signal. In some embodiments, a size of the second gain may be determined by adjusting the component parameters (e.g., sizes of the capacitor and the resistor, etc.) in the low-pass filtering circuit 150. In some embodiments, the component in the low-pass filtering circuit 150 that provides the second gain may be provided at a first stage of the low-pass filtering circuit 150, so that the signal is gained before being low-pass filtered.

Several examples of the low-pass filtering circuit 150 are provided below to describe in detail a specific implementation of the low-pass filtering circuit 150.

FIGs. 5A-5B are schematic diagrams illustrating a structure of an exemplary low-pass filtering circuit according to some embodiments of the present disclosure. In some embodiments, the low-pass filtering circuit 150 may include a seventh-order low-pass filter 510, and an input end of the seventh-order low-pass filter 510 is coupled to a high-pass filtering circuit 140 to take a gained EMG signal and a gained detection signal as an input for filtering.

As shown in FIG. 5A, the seventh-order low-pass filter 510 may include operational amplifiers U3A-U3C, resistors R9-R12, R16-R20, and capacitors C11-C15, C17, C19. A positive phase input end of an operational amplifier U3A may be connected to the resistors R9-R11 connected in series. The positive phase input end of the operational amplifier U3A may also be connected to a virtual ground VCC2 through the resistor R12. A negative phase input end of the operational amplifier U3A may be connected to an output end of the operational amplifier U3A through the resistor R16. A power supply end of the operational amplifier U3A is connected to a power supply VCC, and a grounding end of the operational amplifier U3A is grounded. The positive phase input end of the operational amplifier U3B may be connected to the output end of the operational amplifier U3A through the series-connected resistors R16 and R18, and the negative phase input end of the operational amplifier U3B may be connected to the output end of the operational amplifier U3B, and the power end and the grounding end of the operational amplifier U3B are connected in vacuo. The positive phase input end of the operational amplifier U3C may be connected to the output end of the operational amplifier U3B through the series-connected resistors R19-R20, the negative phase input end of the operational amplifier U3C may be connected to the output end of the operational amplifier U3C, and the power end and the grounding end of the operational amplifier U3C are connected in vacuo.

In some embodiments, an amplification subcircuit in the seventh-order low-pass filter 510 may provide a second gain for input signals. In some embodiments, the operational amplifiers U3A-U3C may provide a second gain for the input signals (e.g., the EMG signal and the detection signal after the first gain). A size of the second gain may be correlated to parameters of the resistors R9-R12, R16-R20, and the capacitors C11-C15, C17, C19. In some embodiments, the output end of the operational amplifier U2C may be configured to output an EMG signal and a detection signal that have been low-pass filtered and partially gained (e.g., the second gain).

One end of the capacitor C11 may be connected to a connection point between the resistor R9 and the resistor R10, and the other end of the capacitor C11 may be connected to the virtual ground VCC2. One end of the capacitor C13 may be connected to the connection point between the resistor R10 and the resistor R11, and the other end of the capacitor C13 may be connected to the output end of the operational amplifier U3A. One end of the capacitor C14 may be connected to the positive phase input end of the operational amplifier U3B, and the other end of the capacitor C14 may be connected to the virtual ground VCC2. One end of the capacitor C15 may be connected to the connection point between the resistor R17 and the resistor R18, and the other end of the capacitor C15 may be connected to the output end of the operational amplifier U3B. One end of the capacitor C17 may be connected to the positive phase input end of the operational amplifier U3C, and the other end of the capacitor C17 may be connected to the virtual ground VCC2. One end of the capacitor C19 may be connected to the connection point between the resistor R19 and the resistor R20, and the other end of the capacitor C19 may be connected to the output end of the operational amplifier U3C.

In some embodiments, the seventh-order low-pass filter 510 may use the operational amplifiers U3A-U3C for a low-pass filtering on the detection signal and the EMG signal. In some embodiments, a second cutoff frequency of the seventh-order low-pass filter 510 may be determined by adjusting component parameters of the operational amplifiers U3A-U3C.

Similar to the aforementioned fifth-order high-pass filter 410, in some embodiments, the seventh-order low-pass filter 510 may also be a Chebyshev filter.

In some embodiments, the low-pass filtering circuit 150 may include a passive low-pass filter 520, with an input end of the passive low-pass filter 520 coupled to the high-pass filtering circuit 140 to use the gained EMG signal and the gained detection signal as inputs for filtering.

As shown in FIG. 5B, the passive low-pass filter 520 may include the resistors R1-R2, R8-R9, and the capacitors C2-C3, C12-C13. The resistors R8-R9, R2, and R1 are connected in series sequentially, and one end of each of the capacitors C2-C3, and C12-C13 is connected to the virtual ground VCC2. The other end of the capacitor C12 is connected to the connection point between the resistors R8-R9, the other end of the capacitor C13 is connected to the connection point between the resistors R9 and R2, the other end of the capacitor C2 is connected to the connection point between the resistors R2 and R1, and the other end of the capacitor C3 is connected to the resistor R1.

In some embodiments, one end of the resistor R8 away from the resistor R9 may be configured to receive the gained EMG signal and the gained detection signal, and one end of the resistor R1 away from the resistor R2 may be configured to output the low-pass filtered EMG signal and detection signal. In some embodiments, the passive low-pass filter 520 includes four RC low-pass filtering units. Exemplarily, the passive low-pass filter 520 may include a capacitor C12-resistor R8, a capacitor C13-resistor R9, a capacitor C2-resistor R2, and a capacitor C3-resistor R1. In some embodiments, the passive low-pass filter 520 may use the aforementioned RC low-pass filtering unit for the low-pass filtering on the detection signal and the EMG signal. In some embodiments, the second cutoff frequency of the passive low-pass filter 520 may be determined by adjusting the component parameters in the foregoing RC low-pass filter unit.

In embodiments of the present disclosure, a fact that the passive low-pass filter 520 may not require an additional power supply (e.g., the power supply VCC) allows the passive low-pass filter 520 to introduce a smaller mixed noise, which improves a quality of the EMG signal and the detection signal.

It is noted that instead of providing a gain (e.g., the second gain) to the input signals (e.g., the EMG signal and the detection signal after the first gain), the passive low-pass filter 520 may utilize the amplification subcircuit in the aforementioned active bandpass filter 420 to provide the first gain to the EMG signal and the detection signal to achieve the gain function. In some embodiments, the gain function may also be realized by providing the second gain by disposing an independent active amplification subcircuit at the input end of the passive low-pass filter 520. Moreover, the foregoing variety of filters (e.g., the fifth-order high-pass filter 410, the active bandpass filter 420, the seventh-order low-pass filter 510, the passive low-pass filter 520) are examples, and types, structures, and the parameters of the components of the filters may be adjusted according to filtering requirements as well as gain requirements.

In some embodiments, a secondary gain circuit (not shown in FIG. 3) may also be provided between the electrode 110 and the high-pass filtering circuit 140, and the secondary gain circuit provides a secondary gain that is less than the first gain and the second gain.

In some embodiments, the secondary gain circuit may be an amplification subcircuit independently disposed to provide the secondary gain to the EMG signal and the detection signal to achieve the gain function. In some embodiments, the secondary gain may provide a partial gain for the EMG signal and the detection signal. In some embodiments, a size of the secondary gain may be determined by adjusting the component parameters in the secondary gain circuit (e.g., a size of the capacitor resistor, etc.).

In some embodiments, the secondary gain circuit may provide a secondary gain of 1-1000 times. In some embodiments, the secondary gain circuit is located in a former stage compared to the other amplification subcircuits, and the gain provided by the secondary gain circuit may be less than the gain provided by the other amplification subcircuits. In some embodiments, the secondary gain circuit may provide 10 times the secondary gain.

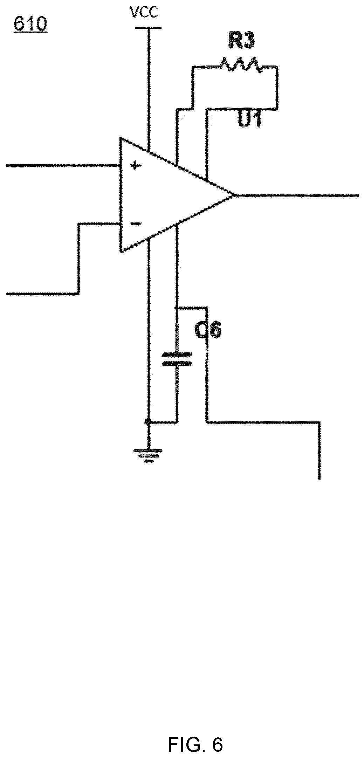

FIG. 6 is a schematic diagram illustrating a structure of an exemplary secondary gain circuit 610 according to some embodiments of the present disclosure. As shown in FIG. 6, in some embodiments, the secondary gain circuit 610 may include an operational amplifier U1, and a positive phase input end and a negative phase input end of the operational amplifier U1 are connected to at least one electrode, respectively, to receive an EMG signal and a detection signal as input signals. A power supply end of the operational amplifier U1 is connected to a power supply VCC, and a grounding end of the operational amplifier U1 is grounded. A reference end of the operational amplifier U1 is connected to the virtual ground VCC2, and an output end of the operational amplifier U1 is configured to output a gained signal, and a value of the gained signal may be equal to a value of a difference between the output end and the reference end of the operational amplifier U1.

A first gain control end and a second gain control end of the operational amplifier U1 may be connected through a resistor R3. In some embodiments, there exists a correspondence between a resistance value of the resistor R3 and the magnification times of the operational amplifier U1. In some embodiments, a secondary gain provided by the secondary gain circuit 610 for the input signal may be determined by adjusting the resistance value of the resistor R3. Exemplarily, by increasing the resistance value of resistor R3, the secondary gain may be reduced; conversely, by reducing the resistance value of resistor R3, the secondary gain may be increased.

In some embodiments, the operational amplifier U1 may be an amplifier such as a differential amplifier or an instrumentation amplifier, so that the operational amplifier U1 has a property of a high input impedance capable of reducing variations in the input signal of the circuit when connected. In some embodiments, the secondary gain circuit 610 has the high input impedance, which reduces the variation of the signal that occurs when the circuit is connected, allowing for the detection signal collected to be more stable.

Continuing with reference to FIG. 3, in some embodiments, the signal measuring device 100 may further include: an ADC 160 connected to the low-pass filtering circuit 150, and the ADC 160 is configured to perform an analog-to-digital conversion on the filtered EMG signal and the filtered detection signal.

In some embodiments, the ADC 160 may sample the EMG signal and the detection signal according to a sampling rate to generate digital signals corresponding to the EMG signal and the detection signal. In some embodiments, the sampling rate of the ADC 160 may be set in conjunction with a frequency of an AC excitation source (i.e., a first frequency). Specifically, to collect the EMG signal and the detection signal at a target frequency, the sampling rate of the ADC 160 may be greater than two times a third frequency. The third frequency is a greater frequency between the first frequency and the greatest target frequency of the EMG signal. To minimize errors, in some embodiments, the sampling rate may be four times or more of the third frequency. It should be noted that due to the great count of channels of the signal measuring device 100, it is difficult for most of the circuits to support the very high sampling rate of the plurality of channels, so it is not suitable to have a too high single-channel sampling rate and first frequency of the AC excitation source. For example, the first frequency may be 1 kHz and the single-channel sampling rate may be 8 kHz.

As the EMG signal and the detection signal are processed at the same time, the EMG signal and the detection signal may be mixed together. In some embodiments, the signal measuring device 100 may also include a digital circuit (not shown in FIG. 3) located at the output end of the ADC 160 to extract and separate the analog-to-digital converted EMG signal from the analog-to-digital converted detection signal. In some embodiments, the signal measuring device 100 may further include a second low-pass filtering circuit and a second high-pass filtering circuit located at the output end of the ADC 160. The second low-pass filtering circuit may be configured to extract the analog-to-digital converted EMG signal, and the second high-pass filtering circuit may be configured to extract the analog-to-digital converted detection circuit.

In the embodiments of the present disclosure, by separately extracting the EMG signal and the detection signal, it is possible to subsequently and independently use information including a quality of the detection signal on the EMG signal, and a fitting state between the electrode and the human body to perform more accurate evaluations.

In some embodiments, the electrodes 110 may include a first electrode and a second electrode to collect a first EMG signal and a second EMG signal in the EMG signals, respectively. The AC excitation source 120 includes two excitation sources (e.g., a first AC excitation source and a second AC excitation source) connected to the first electrode and the second electrode, respectively, and provide two excitation signals (e.g., a first excitation signal and a second excitation signal), respectively, to generate a first detection signal and a second detection signal in the detection signal. Correspondingly, the gain circuit 130 includes the differential amplifier that performs differential amplification on the first EMG signal and the second EMG signal, respectively, on the first detection signal and the second detection signal.

In some embodiments, the frequencies of the first excitation signal and the second excitation signal provided by the AC excitation source 120 may be different, so as to make the frequencies of the generated first detection signal and the second detection signal different. In some embodiments, the first electrode and the second electrode may have a great distance (e.g., greater than or equal to 5 cm) from each other so that a closed-loop circuit through which the first excitation signal and the second excitation signal flow may pass through different human tissues, resulting in different frequencies of the first detection signal and the second detection signal corresponding to the first electrode and the second electrode, respectively, which is configured to reflect body composition information of the human body.

In some embodiments, a plurality of frequencies may be included in the first excitation signal and the second excitation signal provided by the AC excitation source 120, respectively, such that a plurality of frequencies are also included in both of the first detection signal and the second detection signal. In this way, impedance information of the human body at different frequencies may be obtained by operationally amplifying the first detection signal and the second excitation signal, which is configured to reflect the body composition information of the human body.

In some embodiments, the first excitation signal and the second excitation signal provided by the AC excitation source 120 may also be of the same frequency, so that the generated first detection signal and the generated second detection signal are also of the same frequency, thereby allowing for a simultaneous collection of a plurality of detection signals and a plurality of EMG signals for timely evaluation and feedback of the signals.

In embodiments of the present disclosure, the detection signal reflecting the contact impedance may be collected at the same time when collecting the EMG signal to evaluate the EMG signal, and subsequently, the contact impedance may be adjusted to be suitable so that the EMG signal with a higher quality is able to be collected, and then a gain may be provided to the EMG signal and the detection signal. In this way, the strength and the quality of the EMG signal may be enhanced even when the signal strength is small, thereby ensuring subsequent identification and processing of the EMG signal.

In some embodiments, the signal measuring device 100 may be applied to a wearable device. In some embodiments, the wearable device may include a smart bracelet, smart footwear, smart glasses, a smart helmet, a smart watch, a smart garment, a smart backpack, a smart accessory, etc., or any combination thereof. Taking a smart garment (e.g., a fitness garment or sportswear) as an example, the signal measuring device 100 may obtain physiological signals collected by any two electrodes or two electrodes with a specific association (e.g., two electrodes contact with the same muscle to receive the EMG signal of the same muscle), and evaluate, based on a difference between the physiological signals collected by the any two electrodes or the two electrodes with the specific association, the quality of the physiological signals, or evaluate the fitting state between the any two electrodes or the two electrodes and the human body.

In some embodiments, the signal measuring device 100 may also perform a wearing state analysis, a warm-up state analysis, a size fit analysis, a garment life analysis, a motion monitoring, a skin state analysis, a body composition analysis, etc.

Three exemplary signal measuring devices are provided below to describe specific implementations of the signal measuring devices.

### Example 1:

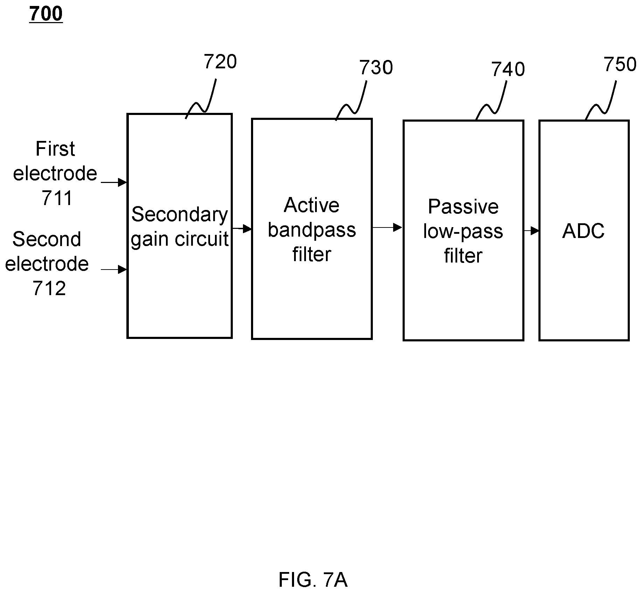

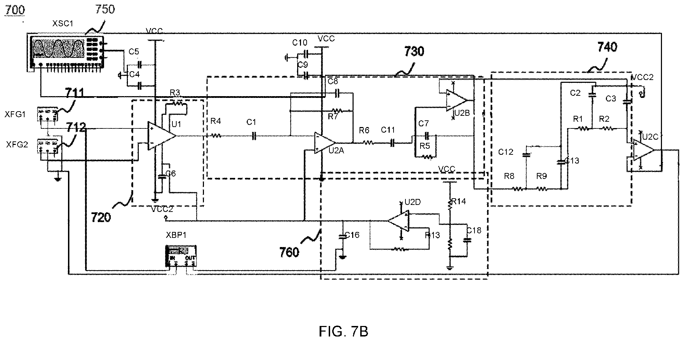

FIG. 7A is a block diagram illustrating a circuit structure of a signal measuring device of Example 1 according to some embodiments of the present disclosure. FIG. 7B is a schematic diagram illustrating the circuit structure of the signal measuring device of Example 1 according to some embodiments of the present disclosure. As shown in FIGS. 7A-7B, this embodiment provides a signal measuring device 700 that includes: at least one electrode, an AC excitation source (not shown in the figures), a secondary gain circuit 720, an active bandpass filter 730, a passive low-pass filter 740, and an ADC 750. The at least one electrode may include a first electrode 711 and a second electrode 712. The first electrode 711 and the second electrode 712 are connected to a secondary gain circuit 720, respectively, and the secondary gain circuit 720, the active bandpass filter 730, the passive low-pass filter 740, and the ADC 750 are connected sequentially.

In some embodiments, the first electrode 711 and the second electrode 712 (a function generator XFG1 and a function generator XFG2 as shown in FIG. 7B) may collect an EMG signal and send the EMG signal to the secondary gain circuit 720, respectively, and the AC excitation source may provide an excitation signal at a first frequency to generate a detection signal reflecting a contact impedance between the first electrode 711, the second electrode 712, and a human body. In some implementations, structures and functions of the first electrode 711 and the second electrode 712 may be the same as or similar to the electrode 110 described above. Specific implementations of the electrodes may be referred to in other parts of the present disclosure, e.g., FIGs.1-6 and the descriptions thereof.

An input end of the secondary gain circuit 720 receives the EMG signal and the detection signal and provides a secondary gain thereto. In some embodiments, the secondary gain circuit 720 may be of the same or similar structure and function as the secondary gain circuit 610 described above. Specific descriptions of the secondary gain circuit 720 may be referred to in other parts of the present disclosure, such as FIG. 6 and the descriptions thereof.

The active bandpass filter 730 receives the EMG signal and the detection signal from the secondary gain circuit 720, extracts the EMG signal and the detection signal according to a preset bandwidth frequency range to filter out a low frequency noise in the signals, and avoids subsequently process of providing a gain for the low frequency noise, thereby ensuring that the signals do not exceed a measurement range of the signal measuring device 700.

In some embodiments, an amplification subcircuit in the active bandpass filter 730 may also provide a first gain for the filtered EMG signal and the detection signal. Both the secondary gain circuit 720 and the amplification subcircuit may implement the gain function, providing a secondary gain and the first gain for the EMG signal and the detection signal to improve the quality of the signals. In some implementations, the active bandpass filter 730 may have the same or similar structure and function as the active bandpass filter 420 described above. Specific descriptions of the active bandpass filter 730 may be found in other parts of the present disclosure, such as FIG. 4B and the descriptions thereof.

In some embodiments, the passive low-pass filter 740 may receive the EMG signal and the detection signal from the active bandpass filter 730, and after the EMG signal and the detection signal are gained, high-frequency noises in the EMG signal and the detection signal are filtered out based on a second cutoff frequency to avoid introducing the high-frequency noise during the gain process, so as to improve a signal quality. In some embodiments, the passive low-pass filter 740 may have the same or similar structure and function as the passive low-pass filter 520 described above. Specific descriptions of the active bandpass filter 730 may be found in other parts of the present disclosure, such as FIG. 5B and the descriptions thereof.

In some embodiments, the ADC 750 (such as an ADC XSC1 shown in FIG. 7B) may receive the EMG signal and the detection signal from the passive low-pass filter 740 and perform an analog-to-digital conversion thereon. In some embodiments, the EMG signal and the detection signal after the analog-to-digital conversion may also be processed by a filter to extract the EMG signal and the detection signal, respectively, for subsequent evaluation of the EMG signal and the detection signal. In some embodiments, the structure and function of the ADC 750 may be the same or similar to the ADC 160 described above. Specific descriptions of the ADC 750 may be referred to in other parts of the present disclosure, e.g., FIG. 1 - FIG. 6 and descriptions thereof.

Referring to FIG. 7B, in some embodiments, the assembly of the passive low-pass filter 740 and ADC 750 may also be provided with a follower U2C, and the follower U2C may be configured to maintain an output voltage or current stable, isolating the passive low-pass filter 740 from the ADC 750. A positive phase input end of the follower U2C is connected to the output end of the passive low-pass filter 740 to receive the EMG signal and the detection signal. A negative phase input end of the follower U2C is connected to an output end of the follower U2C, and a power supply end and a grounding end of the follower U2C are connected.

Referring to FIG. 7B, in some embodiments, the signal measuring device 700 may further include a spectrum analyzing device XBP1 to measure a frequency response curve of the signal measuring device 700. One end of the spectrum analyzing device XBP1 is connected to the function generator XFG1 and the function generator XFG2 (i.e., the first electrode and the second electrode), respectively, and the other end of the spectrum analyzing device XBP1 is connected to the output of the follower U2C.

Continuing to refer to FIG. 7B, in some embodiments, the signal measuring device 700 may further include a power supply circuit 760, the power supply circuit 760 may be connected to a power supply VCC, and an output of the power supply circuit 760 may be connected to a virtual ground VCC2 connected to provide a power to the virtual ground VCC2. In some embodiments, when the voltage of the power supply VCC is 3.3 V, the power supply circuit 760 may provide a power supply of 1.65 V or other power supply voltage within a dynamic range to the virtual ground VCC2.

In some embodiments, the power supply circuit 760 may include an operational amplifier U2D, a positive phase input end of the operational amplifier U2D is connected to the power supply VCC through a resistor R14, and the positive phase input end of the operational amplifier U2D is also grounded via a resistor R13 and a capacitor C18 connected in parallel. A negative phase input end of the operational amplifier U2D is connected to an output end of the operational amplifier U2D through a resistor R15, and the output end of the operational amplifier U2D is grounded through a capacitor C16. Exemplarily, an impedance value of the resistors R13- R14 may be 10 kΩ, an impedance value of the resistor R15 may be 300 Ω, a capacitance value of the capacitor C16 may be 10 µF, a capacitance value of the capacitor C18 may be 10 µF, and a specification of the operational amplifier U2D may be TLC2274ACPW.

### Example 2:

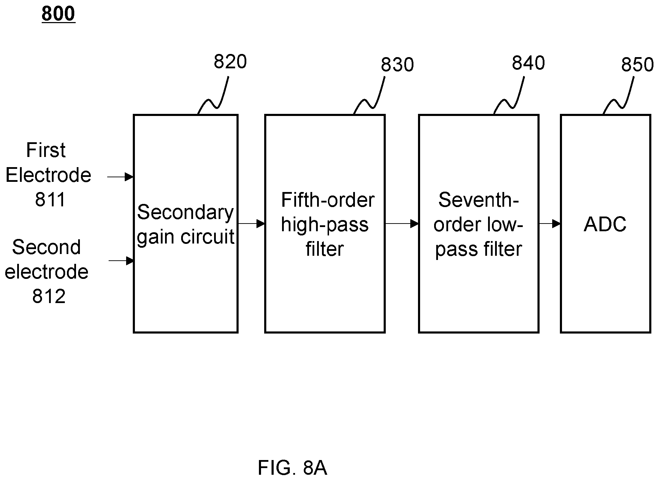

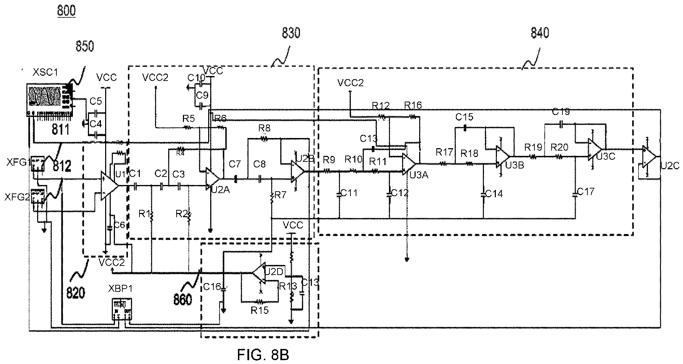

FIG. 8A is a block diagram illustrating a circuit structure of an exemplary signal measuring device of Example 2 according to some embodiments of the present disclosure; and FIG. 8B is a schematic diagram illustrating the circuit structure of the exemplary signal measuring device of Example 2 according to some embodiments of the present disclosure.

As shown in FIGs. 8A-8B, this embodiment provides a signal measuring device 800 that may include: at least one electrode, an AC excitation source (not shown in the figures), a secondary gain circuit 820, a fifth-order high-pass filter 830, a seventh-order low-pass filter 840, and an ADC 850. The electrodes may include a first electrode 811 and a second electrode 812. The first electrode 811 and the second electrode 812 are connected to the secondary gain circuit 820, respectively. The secondary gain circuit 820, the fifth order high-pass filter 830, the seventh order low-pass filter 840, and the ADC 850 are connected sequentially.

In some embodiments, the first electrode 811 and the second electrode 812, the AC excitation source, the secondary gain circuit 820, and the ADC 850 in this embodiment are similar to the first electrode 711 and the second electrode 712, the AC excitation source, the secondary gain circuit 720, and the ADC 750 in the above-described Example 1. The specific descriptions may be referred to the above Example 1, which are not repeated here.

In some embodiments, the fifth-order high-pass filter 830 may receive the EMG signal and the detection signal from the secondary gain circuit 820, filter low frequency noises in the EMG signal and the detection signal based on a first cutoff frequency to avoid subsequently process of providing a gain for the low frequency noise, so as to ensure that the signals do not exceed a measurement range of the signal measuring device 800. In some embodiments, an amplification subcircuit in the fifth-order high-pass filter 830 may also provide a first gain for the filtered EMG signal and detection signal.

In some embodiments, the structure and function of the fifth-order high-pass filter 830 may be the same or similar to the fifth-order high-pass filter 410 described above. Specific descriptions of the fifth-order high-pass filter 830 may be referred to in other parts of the present disclosure, such as FIG. 4A and the descriptions thereof.

In some embodiments, the seventh-order low-pass filter 840 may receive the EMG signal and the detection signal from the fifth-order high-pass filter 830, and after the EMG signal and the detection signal are gained, high-frequency noises in the EMG signal and the detection signal are filtered out based on a second cutoff frequency. In this way, the introduction of the high-frequency noise during the gain process may be avoided to improve the quality of the signal. In some embodiments, the amplifier subcircuit in the seventh-order low-pass filter 840 may also provide a second gain for the EMG signal and the detection signal before filtering. By filtering after gain, the mixed signal generated in the gain may be avoided, so as to improve the quality of the EMG signal and the detection signal.

In some implementations, the seventh-order low-pass filter 840 may have the same or similar structure and function as the seventh-order low-pass filter 510 described above. Specific descriptions of the seventh-order low-pass filter 840 may be found in other parts of the present disclosure, such as FIG. 5A and the descriptions thereof.

In some embodiments, the secondary gain circuit 820, the amplification subcircuit in the fifth-order high-pass filter 830, and the amplification subcircuit in the seventh-order low-pass filter 840 may all implement the gain function to provide the EMG signal and the detection signal with a secondary gain, the first gain, and the second gain to improve the quality of the signals.

Referring to FIG. 8B, in some embodiments, a follower U2C may also be disposed between the seventh-order low-pass filter 840 and the ADC 850. The follower U2C in this embodiment is similar to the follower U2C in the above-described Example 1. The specific implementation may be referred to the above-described Example 1, which are not repeated here.

In some embodiments, the signal measuring device 800 may further include a spectrum analysis device XBP1 and a power supply circuit 860. The spectrum analysis device XBP1 and the power supply circuit 860 in this embodiment is similar to the spectrum analysis device XBP1 and the power supply circuit 760 in the above-described Example 1, and the specific implementation may be referred to in the above-described Example 1, which are will not repeated here.

### Example 3:

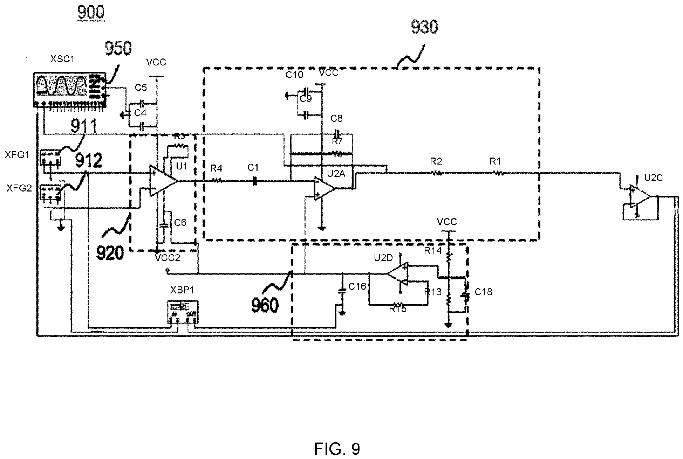

FIG. 9 is a schematic diagram illustrating a circuit structure of a bandpass gain signal measuring device of Example 3 according to some embodiments of the present disclosure.

As shown in FIG. 9, this embodiment provides a bandpass gain signal measuring device 900 that includes: at least one electrode, an AC excitation source (not shown in the figure), a secondary gain circuit 920, a bandpass gain circuit 930, and an ADC 950. The at least one electrode may include a first electrode 911 and a second electrode 912. The first electrode 911 and the second electrode 912 are connected to the secondary gain circuit 920, respectively, and the secondary gain circuit 920, the bandpass gain circuit 930, and the ADC 950 are connected sequentially.

In some embodiments, the first electrode 911 and the second electrode 912, the AC excitation source, the secondary gain circuit 920, and the ADC 950 in this embodiment are similar to the first electrode 711 and the second electrode 712, the AC excitation source, the secondary gain circuit 720, and the ADC 750 in the above Example 1. The specific implementation may be referred to in the above Example 1, which are not repeated herein.

In some embodiments, the bandpass gain circuit 930 may receive an EMG signal and a detection signal from the secondary gain circuit 920, extract the EMG signal and the detection signal according to a third bandwidth frequency range, and simultaneously filter a low-frequency noise and a high-frequency noise in the signals, thereby improving the quality of the EMG signal and the detection signal to a certain extent. In some embodiments, the third bandwidth frequency range may include a frequency range in which the EMG signal and the detection signal are located. In some embodiments, an amplification subcircuit in the bandpass gain circuit 930 may also provide a third gain for the filtered EMG signal and detection signal.

Referring to FIG. 9, in some embodiments, the bandpass gain circuit 930 may include an operational amplifier U2A, resistors R1-R2, R4, R7, and capacitors C1, C8. A positive phase input end of the operational amplifier U2A may be connected to a virtual ground VCC2. A negative phase input end of the operational amplifier U2A may be connected to the series-connected capacitor C1 and the resistor R4, and the negative phase input end of the operational amplifier U2A may be connected to an output end of the operational amplifier U2A through the parallel-connected capacitor C8 and the resistor R7. A power supply end of the operational amplifier U2A is connected to a power supply VCC, and a grounding end of the operational amplifier U2A is grounded. The series-connected resistors R1-R2 are set at the output end of the operational amplifier U2A.

In some embodiments, the operational amplifier U2A of the bandpass gain circuit 930 may provide a third gain for input signals (e.g., the EMG signal and the detection signal). A size of the third gain may be related to parameters of the resistors R4 and R7, and the capacitors C1 and C8. In some embodiments, the third bandwidth frequency range of the bandpass gain circuit 930 may also be determined by adjusting component parameters of the aforementioned resistors R4 and R7, and capacitors C1 and C8.

Exemplarily, a capacitance value of the capacitor C1 may be 1 µF, and the capacitance value of the capacitor C8 may be 33 nF. Impedance values of the resistors R1-R2 may be 500 Ω, the impedance value of the resistor R4 may be 680 Ω, and the impedance value of the resistor R7 may be 30 kΩ. The power supply VCC may be 3.3V, and specifications of the operational amplifiers U2A and U2B may be TLC2274ACPW.

In some embodiments, the amplification subcircuits in the secondary gain circuit 920 and the bandpass gain circuit 930 may implement a gain function, so as to provide the secondary gain and the third gain for the EMG signal and the detection signal to improve the quality of the signals.

Referring to FIG. 9, in some embodiments, a follower U2C may also be disposed between the bandpass gain circuit 930 and the ADC 950. The follower U2C in this embodiment is similar to the follower U2C in the above-described Example 1. The specific implementation may be referred to in the above-described Example 1, which is not repeated herein.

In some embodiments, the signal measuring device 900 may further include a spectrum analysis device XBP1 and a power supply circuit 960. The spectrum analysis device XBP1 and the power supply circuit 960 in this embodiment is similar to the spectrum analysis device XBP1 and the power supply circuit 760 in the above-described Example 1. The specific implementation may be referred to in the above-described Example 1, which is not repeated here.

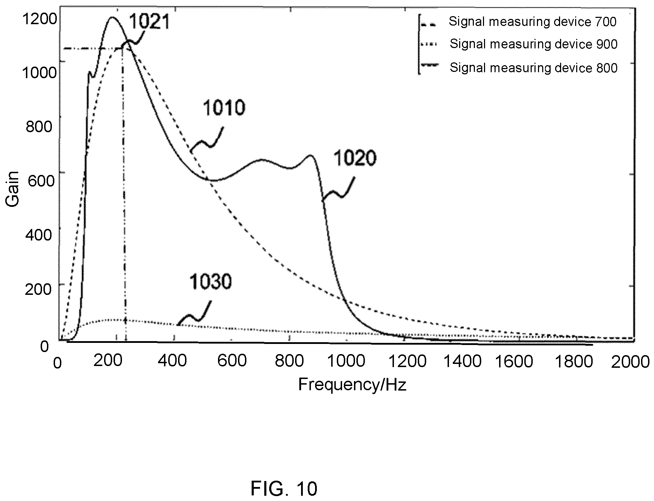

FIG. 10 is a diagram illustrating frequency response curves of the signal measuring devices in FIG. 7B, FIG. 8B and FIG. 9. As shown in FIG. 10, a curve 1010 is a frequency response curve of the signal measuring device 700 shown in FIG. 7B; a curve 1020 is the frequency response curve of the signal measuring device 800 shown in FIG. 8B; and a curve 1030 is the frequency response curve of the signal measuring device 900 shown in FIG. 9.

Referring to FIG. 10, the curve 1010 has a peak (a peak 1021 shown in FIG. 10). The peak 1021 is distributed around 210 Hz, which corresponds to a gain of about 1050 times. The curve 1020 is similar to the curve 210 shown in FIG. 2A above, with a double peak. An overall amplitude of curve 1030 is smaller, which reflects that the signal measuring device 900 provides a smaller gain for an EMG signal and a detection signal.

Referring to FIG. 10, SNRs for all three gains of the signal measuring device 800 are improved compared to the signal measuring device 700 and the signal measuring device 900. Exemplarily, compared to the signal measuring device 700, the signal measuring device 800 has an improved MA SNR of 10,285 times, an improved IF SNR of 45.8 times, and an improved mixed SNR of 87.7 times. Compared to the signal measuring device 900, the signal measuring device 800 has an improved MA SNR of 75080.5 times, an improved IF SNR of 82.44 times, and an improved mixed SNR of 1315.5 times.

Beneficial effects brought about by the embodiments of the present disclosure include, but are not limited to: (1) the detection signal reflecting the contact impedance may be simultaneously collected during the collection of the EMG signal, so that appropriate subsequent adjustment of the contact impedance may be performed to ensure that the at least one electrode fits the human body better, and that the EMG signal of a higher quality may be collected; and (2) by setting a gain circuit, the collected EMG signal and detection signal are provided with a suitable gain, so that the strength and the quality of the EMG signal and the detection signal may be improved, and the signal measuring device may accurately carry out monitoring.

The basic concepts have been described above, and it is apparent to those skilled in the art that the foregoing detailed disclosure is intended as an example only and does not constitute a limitation of the present disclosure. Although not expressly, stated herein, those skilled in the art may make various modifications, improvements and amendments to the present disclosure. Such modifications, improvements, and amendments are suggested in the present disclosure, so such modifications, improvements, and amendments remain within the spirit and scope of the exemplary embodiments of the present disclosure.

Also, the present disclosure uses specific words to describe embodiments of the present disclosure. Such as "an embodiment," "one embodiment," and/or "some embodiment" means a feature, structure, or characteristic associated with at least one embodiment of the present disclosure. Accordingly, it should be emphasized and noted that the "one embodiment" or "an embodiment" or "an alternative embodiment" in different places in the present disclosure do not necessarily refer to the same embodiment. In addition, certain features, structures, or characteristics in one or more embodiments of the present disclosure may be suitably combined.

Additionally, unless expressly stated in the claims, the order of the processing elements and sequences, the use of numerical letters, or the use of other names as described in the present disclosure are not intended to limit the order of the processes and methods of the present disclosure. While some embodiments of the present disclosure that are currently considered useful are discussed in the foregoing disclosure by way of various examples, it should be appreciated that such details serve only illustrative purposes, and that additional claims are not limited to the disclosed embodiments. Rather, the claims are intended to cover all amendments and equivalent combinations that are consistent with the substance and scope of the embodiments of the present disclosure. For example, although the implementation of various components described above may be embodied in a hardware device, it may also be implemented as a software only solution, e.g., on an existing server or mobile device.

Similarly, it should be noted that in order to simplify the presentation of the disclosure of the present disclosure, and thereby aiding in the understanding of one or more embodiments of the present disclosure, the foregoing descriptions of embodiments of the present disclosure sometimes group multiple features together in a single embodiment, accompanying drawings, or a description thereof. However, the manner of disclosure does not imply that the objects of the present disclosure require more features than those mentioned in the claims. Rather, claimed subject matter may lie in less than all features of a single foregoing disclosed embodiment.

Some embodiments use counts describing the count of components, attributes, and it should be understood that such counts used in the description of embodiments are modified in some examples by the modifiers "approximately," "nearly," or "substantially." Unless otherwise noted, the terms "about," "approximate," or "approximately" indicates that a ±20% variation in the stated count is allowed. Correspondingly, in some embodiments, the numerical parameters used in the present disclosure and claims are approximations, and the approximations are subject to change depending on the expected characteristics of individual embodiments. In some embodiments, the numerical parameters should take into account the specified count of valid digits and utilize a general digit retention method. While the numerical domains and parameters used to confirm the breadth of their ranges in some embodiments of the present disclosure are approximations, in specific embodiments such values are set to be as precise as possible within a feasible range.

For each of the patents, patent applications, patent application disclosures, and other materials cited in the present disclosure, such as articles, books, specification sheets, publications, documents, etc., the entire contents of which are hereby incorporated herein by reference. Application history documents that are inconsistent with or conflict with the contents of the present disclosure are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appended to the present disclosure and those set forth herein, the descriptions, definitions and/or use of terms in the present disclosure shall prevail.

Finally, it should be understood that the embodiments described in the present disclosure are only used to illustrate the principles of the embodiments of the present disclosure. Other deformations may also fall within the scope of the present disclosure. As such, alternative configurations of embodiments of the present disclosure may be viewed as consistent with the teachings of the present disclosure as an example, not as a limitation. Correspondingly, the embodiments of the present disclosure are not limited to the embodiments expressly presented and described herein.

## Claims

1. A signal measuring device, comprising:
at least one electrode configured to contact with a human body to collect an electromyographic (EMG) signal of the human body in a target frequency range;
an alternating current (AC) excitation source electrically connected to the at least one electrode, wherein the AC excitation source is configured to provide an excitation signal at a first frequency to generate a detection signal reflecting a contact impedance between the at least one electrode and the human body; and
a gain circuit configured to provide a gain for the EMG signal and the detection signal.

2. The signal measuring device of claim 1, wherein the target frequency range includes a range of 20 Hz-400 Hz, and the first frequency is not less than 250 Hz.

3. The signal measuring device of claim 2, wherein a difference between the first frequency and any integer multiple of 50 Hz is not less than 1 Hz; or a difference between the first frequency and any integer multiple of 60 Hz is not less than 1 Hz.

4. The signal measuring device of claim 1, wherein the first frequency is higher than the target frequency range.

5. The signal measuring device of claim 1, wherein the gain of the gain circuit for the EMG signal is not less than 1/10 of the gain for the detected signal and is not greater than 10 times the gain for the detected signal.

6. The signal measuring device of claim 5, wherein a frequency response of the gain circuit has one or more peaks in a first frequency range, the first frequency range being higher than 50 Hz and lower than the first frequency.

7. The signal measuring device of claim 5, wherein a ratio of the gain of the gain circuit at 100 Hz to the gain of the gain circuit at 10 Hz is a first signal-to-noise ratio (SNR), and the first SNR is not less than 4.

8. The signal measuring device of claim 5, wherein the ratio of the gain of the gain circuit at 100 Hz to the gain of the gain circuit at 50 Hz is a second SNR, and the second SNR is not less than 2.

9. The signal measuring device of claim 5, wherein the gain circuit further includes a high-pass filtering circuit configured to filter the EMG signal and the detection signal.

10. The signal measuring device of claim 9, wherein the gain circuit further includes a low-pass filtering circuit connected to the high-pass filtering circuit, and the low-pass filtering circuit is configured to filter high-frequency noises in the EMG signal and the detection signal; and
the signal measuring device further includes an analog-to-digital converter (ADC) configured to perform an analog-to-digital conversion on the filtered EMG signal and the filtered detection signal.

11. The signal measuring device of claim 10, wherein the ADC has a sampling rate greater than two times a third frequency, the third frequency being the greater of the first frequency and a frequency of the EMG signal.

12. The signal measuring device of claim 10, wherein a ratio of the gain of the gain circuit at 100 Hz to the gain of the gain circuit at a second frequency is a third SNR, and the second frequency corresponds to 1/2 of a sampling rate of the ADC, and the third SNR is not less than 10.

13. The signal measuring device of claim 10, wherein the high-pass filtering circuit includes an active bandpass filter configured to provide a first gain for the EMG signal and the detection signal; and
the low-pass filtering circuit includes a passive low-pass filter coupled to the active bandpass filter.

14. The signal measuring device of claim 10, wherein the high-pass filtering circuit includes a fifth-order high-pass filter, an input end of the fifth-order high-pass filter receives the EMG signal and the detection signal, and the fifth-order high-pass filter is configured to provide a first gain for the EMG signal and the detection signal; and
the low-pass filtering circuit includes a seventh-order low-pass filter coupled to the fifth-order high-pass filter, and the seventh-order low-pass filter is configured to provide a second gain for the EMG signal and the detection signal.

15. The signal measuring device of claim 14, wherein the fifth-order high-pass filter and the seventh-order low-pass filter are Chebyshev filters.

16. The signal measuring device of any one of claims 13-15, wherein a secondary gain circuit is disposed between the at least one electrode and the high-pass filtering circuit, the secondary gain circuit provides a secondary gain, and the secondary gain is less than the first gain and a second gain.

17. The signal measuring device of claim 1, wherein the at least one electrode includes a first electrode and a second electrode configured to collect a first EMG signal and a second EMG signal of the EMG signal, respectively;
the AC excitation source includes two excitation sources connected to the first electrode and the second electrode, respectively, and provides two excitation signals to generate a first detection signal and a second detection signal of the detection signal, respectively; and
the gain circuit includes a differential amplifier that performs differential amplification on the first EMG signal and the second EMG signal, and the first detection signal and the second detection signal, respectively.

18. The signal measuring device of claim 1, wherein the gain circuit provides the gain to the EMG signal and the detection signal at the same time; or the gain circuit provides the gain to the EMG signal and the detection signal at different time periods, respectively.

19. The signal measuring device of claim 1, wherein the signal measuring device collects the EMG signal and the detection signal at the same time; or the signal measuring device collects the EMG signal and the detection signal at different time periods, respectively.

20. The signal measuring device of claim 1, wherein the signal measuring device is a wearable device.
